# EUROPEAN PATENT SPECIFICATION

(11) **EP 4 357 781 B1**
(45) Date of publication and mention of the grant of the patent: **20.05.2026**
(21) Application number: 22825054.4
(22) Date of filing: 16.06.2022
(51) Int. Cl.: G01N 33/569, G01N 33/577, C07K 14/005

(54) **SARS-COV-2 IMMUNOASSAY METHOD AND IMMUNOASSAY KIT**
SARS-COV-2-IMMUNTESTVERFAHREN UND IMMUNTESTKIT
PROCÉDÉ DE DOSAGE IMMUNOLOGIQUE DU SARS-COV-2 ET KIT DE DOSAGE IMMUNOLOGIQUE

(30) Priority: 16.06.2021 JP 2021100123
(43) Date of publication of application: 24.04.2024
(73) Proprietor: Sekisui Medical Co., Ltd., Tokyo 103-0027 (JP)
(72) Inventor: HIROTA Jiro, Tokyo 103-0027 (JP); UNO Satoru, Tokyo 103-0027 (JP); OCHIAI Yasushi, Tokyo 103-0027 (JP); ITO Shizuka, Tokyo 103-0027 (JP); WATANABE Keisuke, Tokyo 103-0027 (JP); OKUYAMA Shinya, Tokyo 103-0027 (JP); ASAI Tomohide, Tokyo 103-0027 (JP)
(74) Representative: Ter Meer Steinmeister & Partner
(86) International application number: PCT/JP2022/024134
(87) International publication number: WO 2022/265066

(56) References cited:
- WO-A1-2021/181994
- WO-A1-2022/004622
- CN-A- 111 748 032
- CN-A- 112 903 996
- JP-B1- 6 960 508
- US-A1- 2009 280 507
- YAMAOKA YUTARO ET AL: "Highly specific monoclonal antibodies and epitope identification against SARS-CoV-2 nucleocapsid protein for antigen detection tests", CELL REPORTS MEDICINE, vol. 2, no. 6, 1 June 2021 (2021-06-01), pages 100311, XP093269585, ISSN: 2666-3791, DOI: 10.1016/j.xcrm.2021.100311
- ZHANG LI ET AL: "Development of Patient-Derived Human Monoclonal Antibodies Against Nucleocapsid Protein of Severe Acute Respiratory Syndrome Coronavirus 2 for Coronavirus Disease 2019 Diagnosis", FRONTIERS IN IMMUNOLOGY, vol. 11, 13 November 2020 (2020-11-13), XP055807872, DOI: 10.3389/fimmu.2020.595970
- ZHANG L. ET AL: "Development of patient-derived..., supplemental material", 13 November 2020 (2020-11-13), XP093269542, Retrieved from the Internet <URL:https://www.frontiersin.org/journals/immunology/articles/10.3389/fimmu.2020.595970/full#supplementary-material>
- HIROYUKI KOGAKI ET AL: "Novel rapid immunochromatographic test based on an enzyme immunoassay for detecting nucleocapsid antigen in SARS-associated coronavirus", JOURNAL OF CLINICAL LABORATORY, vol. 19, no. 4, 1 January 2005 (2005-01-01), US, pages 150 - 159, XP055716274, ISSN: 0887-8013, DOI: 10.1002/jcla.20070
- TERRY JAMES S ET AL: "Development of a SARS-CoV-2 nucleocapsid specific monoclonal antibody", VIROLOGY, ELSEVIER, AMSTERDAM, NL, vol. 558, 1 February 2021 (2021-02-01), pages 28 - 37, XP086529687, ISSN: 0042-6822, [retrieved on 20210201], DOI: 10.1016/J.VIROL.2021.01.003
- YAMAKAWA, KENTARO: "Development of Rapid Immunochromatographic Enzyme Immunoassay for SARS-COV-2 nucleocapsid antigen", JAPANESE JOURNAL OF MEDICINE AND PHARMACEUTICAL SCIENCE, vol. 77, no. 6, 27 May 2020 (2020-05-27), pages 937 - 944, XP009536599
- TERRY JAMES S.; ANDERSON LORAN BR.; SCHERMAN MICHAEL S.; MCALISTER CARLEY E.; PERERA RUSHIKA; SCHOUNTZ TONY; GEISS BRIAN J.: "Development of a SARS-CoV-2 nucleocapsid specific monoclonal antibody", VIROLOGY, ELSEVIER, AMSTERDAM, NL, vol. 558, 1 February 2021 (2021-02-01), AMSTERDAM, NL , pages 28 - 37, XP086529687, ISSN: 0042-6822, DOI: 10.1016/j.virol.2021.01.003
- ZHANG LI, ZHENG BINYANG, GAO XINGSU, ZHANG LIBO, PAN HONGXIN, QIAO YONG, SUO GUANGLI, ZHU FENGCAI: "Development of Patient-Derived Human Monoclonal Antibodies Against Nucleocapsid Protein of Severe Acute Respiratory Syndrome Coronavirus 2 for Coronavirus Disease 2019 Diagnosis", FRONTIERS IN IMMUNOLOGY, vol. 11, XP055807872, DOI: 10.3389/fimmu.2020.595970
- TIAN XINGUI, MO CHUNCONG, ZHOU LILING, YANG YUJIE, ZHOU ZHICHAO, YOU AIPING, FAN YE, LIU WENKUAN, LI XIAO, ZHOU RONG: "Epitope mapping of severe acute respiratory syndrome-related coronavirus nucleocapsid protein with a rabbit monoclonal antibody", VIRUS RESEARCH, AMSTERDAM, NL, vol. 300, 1 July 2021 (2021-07-01), NL , pages 1 - 7, XP093014610, ISSN: 0168-1702, DOI: 10.1016/j.virusres.2021.198445
- YAMAOKA YUTARO, MIYAKAWA KEI, JEREMIAH SUNDARARAJ STANLEYRAJ, FUNABASHI RIKAKO, OKUDELA KOJI, KIKUCHI SAYAKA, KATADA JUNICHI, WADA: "Highly specific monoclonal antibodies and epitope identification against SARS-CoV-2 nucleocapsid protein for antigen detection tests", CELL REPORTS MEDICINE, vol. 2, 1 June 2021 (2021-06-01), pages 1 - 18, XP055896515, ISSN: 2666-3791, DOI: 10.1016/j.xcrm.2021.100311

## Description

### [Technical Field]

The present invention relates to an immunoassay method and an immunoassay kit for SARS-CoV-2.

Priority is claimed on Japanese Patent Application No. 2021-100123, filed June 16, 2021.

### [Background Art]

SARS-CoV-2 is a virus that causes the coronavirus disease 2019 (COVID-19). SARS-CoV-2 belongs to the family Coronaviridae, which is a family of viruses each having a single-stranded, positive-strand RNA as its genome. Currently, the corona virus disease 2019 is prevalent all over the world, and many cases of infection have been confirmed.

PCR tests and antigen tests are currently being used to detect SARS-CoV-2. The results of the antigen test are available in about 10 minutes after sample collection, so that the antigen test can be performed more easily than the PCR test. On the other hand, a highly sensitive and specific test is required for rapid detection of SARS-CoV-2. However, the antigen test is generally less sensitive than the PCR test. Therefore, there is a need for more sensitive antigen tests.

Bioinformatics has also been used to predict regions in the amino acid sequence of SARS-CoV-2 that can serve as epitopes for immunoassays (Non-Patent Literature 1). However, the production of antibodies that recognize the described regions as epitopes has not actually been implemented.

Further, generally, construction of a sandwich system using two types of antibodies allows antigen tests to measure antigens with high sensitivity. However, when SARS-CoV-2 is an antigen, it is still unclear what kind of antibodies should be combined to construct a sandwich system.

Yamaoka Yutaro et al. ("Highly specific monoclonal antibodies and epitope identification against SARS-CoV-2 nucleocapsid protein for antigen detection tests", CELL REPORTS MEDICINE, vol. 2, no. 6, 1 June 2021) is directed to specific monoclonal antibody antigen detection tests against SARS-CoV-2 nucleocapsid protein, including identification of the epitopes of said monoclonal antibodies, and an immunochromatographic assay using silver amplification technology to select the optimal pair of anti-SARS-CoV-2 monoclonal antibodies using ELISA. The three monoclonal antibodies identified are no. 7, no. 9 and no. 98, which recognize epitopes at amino acid sequences 210-231, 335-348 and 382-397, respectively, of the SARS-CoV-2 nucleocapsid protein which includes 419 amino acids in total.

CN 111 748 032 A relates to the use of different types of monoclonal antibodies against SARS-CoV-2 nucleocapsid protein for the specific detection of SARS-CoV-2.

Zhang Li et al. ("Development of Patient-Derived Human Monoclonal Antibodies Against Nucleocapsid Protein of Severe Acute Respiratory Syndrome Coronavirus 2 for Coronavirus Disease 2019 Diagnosis", FRONTIERS IN IMMUNOLOGY, vol. 11, 13 November 2020) and Zhang L. et al. ("Development of patient-derived..., supplemental material", 13 November 2020) constructed a combinatorial fragment of antigen-binding (Fab)antibody phage library based on peripheral blood-derived from five coronavirus disease 2019 (COVID-19) infected donors. From the library, 159 Fab antibodies were obtained and identified by panning with nucleocapsid protein (NP). Among them, 16 antibodies were evaluated for their binding properties and epitopes recognition. Among these 16 antibodies, two well-paired antibodies were finally screened out for SARS-CoV-2 diagnosis by double-antibody sandwich enzyme-linked immunosorbent assay (ELISA) method.

US 2009/280507 A1 relates to a method for measuring SARS virus nucleocapsid protein (SARS-NP) using first and second antibodies both binding specifically to SARS-NP, wherein the first or second antibody is an antibody recognizing an epitope located in a region (Region C) of amino acid 283 to 422 from the N-terminus of the amino acid sequence of SARS-NP.

Hiroyuki Kogaki et al. ("Novel rapid immunochromatographic test based on an enzyme immunoassay for detecting nucleocapsid antigen in SARS-associated coronavirus", JOURNAL OF CLINICAL LABORATORY, vol. 19, no. 4, 1 January 2005) reports development of a novel rapid immunochromatographic test (RICT) based on the sandwich format enzyme immunoassay (EIA) with an all-in-one device for detecting the native nucleocapsid antigen (N-Ag) of SARS-CoV using monoclonal antibodies (MoAbs), which we produced by immunizing recombinant N-Ag to mice.

CN 112 903 996 A relates to a kit for detecting nCoV-N protein and a method for detecting nCoV-N protein, i.e. a kit and a method for detecting SARS-CoV-2 nucleocapsid protein.

Terry James et al. ("Development of a SARS-CoV-2 nucleocapsid specific monoclonal antibody", VIROLOGY, ELSEVIER, AMSTERDAM, NL, vol. 558, 1 February 2021) discloses a specific detection test for SARS-CoV-2 antigens using specific monoclonal antibodies against SARS-CoV-2 nucleocapsid protein, including identification of the epitopes of said monoclonal antibodies, and also teaches the selection of optimal pairs of anti-SARS-CoV-2 monoclonal antibodies using ELISA.

### [Citation List]

### [Non Patent Literature]

Non-Patent Literature 1 : PATHOGENS AND GLOBAL HEALTH 2020,VOL. 114,NO. 8,463-470 Immunodominant regions prediction of nucleocapsid protein for SARS-CoV-2 early diagnosis: a bioinformatics and immunoinformatics study
Non-Patent Literature 2: Package insert for in-vitro diagnostic reagent "Espline (registered trademark) SARS-CoV-2" (Fujirebio Inc.)
Non-Patent Literature 3: Package insert for in-vitro diagnostic reagent "QuickNavi (registered trademark)-COVID19 Ag" (Denka Company Limited)

### [Summary of Invention]

### [Technical Problem]

An object of the present invention is to provide a SARS-CoV-2 immunoassay kit and a SARS-CoV-2 immunoassay method that enable highly sensitive and rapid immunoassay.

### [Solution to Problem]

The present inventors have made intensive studies to solve the above problems. As a result, the present inventors have found that the above problems can be solved by using two types of monoclonal antibodies or antibody fragments thereof that bind to a peptide fragment having 30 or less consecutive amino acids and respectively recognize different epitopes in the nucleocapsid protein of SARS-CoV-2. Based on this finding, the present invention has been completed.

In the Examples, the present inventors compare the sensitivity of one embodiment of the immunoassay method of the present invention with the sensitivities of Espline (registered trademark) SARS-CoV-2 (Fujirebio Inc.) (Non-Patent Literature 2) and QuickNavi (registered trademark)-COVID19 Ag (Denka Company Limited) (Non-Patent Literature 3), which are commercially available reagents for SARS-CoV-2 antigen detection). The embodiment of the immunoassay method of the present invention exhibits sensitivity comparable or superior to these two reagents with a shorter measurement time.

Specifically, the present invention is as follows and as set out in the appended set of claims.
[1] An in-vitro immunoassay method for assaying SARS-CoV-2 in a biological sample, comprising using two types of monoclonal antibodies or antibody fragments thereof that bind to a peptide fragment having 30 or less consecutive amino acids in a nucleocapsid protein of SARS-CoV-2, wherein the two types of monoclonal antibodies or antibody fragments thereof recognize different epitopes, and wherein the peptide fragment having 30 or less consecutive amino acids is a peptide fragment having an amino acid sequence represented by KQQTVTLLPAADLDDFSKQLQQSMSSA (SEQ ID NO: 1).
[2] The immunoassay method according to [1], wherein the biological sample is a respiratory secretion.
[3] The immunoassay method according to [1] or [2], wherein the two types of monoclonal antibodies or antibody fragments thereof are a first monoclonal antibody or an antibody fragment thereof and a second monoclonal antibody or an antibody fragment thereof, wherein the first monoclonal antibody or the antibody fragment thereof has a labeling substance indirectly or directly bound thereto, and the second monoclonal antibody or the antibody fragment thereof is indirectly or directly bound to a solid phase.
[4] The immunoassay method according to [3], comprising:
   (1) a step of contacting a biological sample with the first monoclonal antibody or the antibody fragment thereof having the labeling substance bound thereto to form a first complex;
   (2) a step of contacting the first complex with the second monoclonal antibody or the antibody fragment thereof to form a second complex; and
   (3) a step of measuring a signal attributable to the labeling substance.
[5] The immunoassay method according to [3] or [4], wherein the first monoclonal antibody or the antibody fragment thereof is a monoclonal antibody or an antibody fragment thereof that recognizes an epitope in the amino acid sequence represented by KQQTVTLLPAADLDFSK (SEQ ID NO: 2), and the second monoclonal antibody or the antibody fragment thereof is a monoclonal antibody or an antibody fragment thereof that recognizes an epitope in the amino acid sequence represented by AADLDDFSKQLQQSMSSA (SEQ ID NO: 3).
[6] The immunoassay method according to any one of [1] to [4], wherein one of the monoclonal antibodies recognizes an epitope in the amino acid sequence represented by KQQTVTLLPAADLDDFSK (SEQ ID NO: 2), while the other monoclonal antibody recognizes an epitope in the amino acid sequence represented by AADLDDFSKQLQQSMSSA (SEQ ID NO: 3).
[7] The immunoassay method according to any one of [1] to [5], wherein the two types of monoclonal antibodies or antibody fragments thereof are, respectively, a monoclonal antibody or an antibody fragment thereof that recognizes an amino acid sequence represented by LLPAA (SEQ ID NO: 26) as an epitope, and a monoclonal antibody or an antibody fragment thereof that recognizes an epitope in an amino acid sequence represented by LDDFSKQLQ (SEQ ID NO: 27).
[8] The immunoassay method according to any one of [1] to [7], which is immunochromatography or ELISA.
[9] The immunoassay method according to any one of [1] to [8], wherein the two types of monoclonal antibodies or the antibody fragments thereof are selected from the group consisting of:
   (1) an antibody or an antibody fragment thereof that comprises a heavy chain variable region comprising CDR1 having an amino acid sequence of SEQ ID NO: 4, CDR2 having an amino acid sequence of SEQ ID NO: 5, and CDR3 having an amino acid sequence of SEQ ID NO: 6, and a light chain variable region comprising CDR1 having an amino acid sequence of SEQ ID NO: 7, CDR2 having an amino acid sequence of SEQ ID NO: 8, and CDR3 having an amino acid sequence of SEQ ID NO: 9;
   (2) an antibody or an antibody fragment thereof that comprises a heavy chain variable region comprising CDR1 having an amino acid sequence of SEQ ID NO: 10, CDR2 having an amino acid sequence of SEQ ID NO: 11, and CDR3 having an amino acid sequence of SEQ ID NO: 12, and a light chain variable region comprising CDR1 having an amino acid sequence of SEQ ID NO: 13, CDR2 having an amino acid sequence of SEQ ID NO: 14, and CDR3 having an amino acid sequence of SEQ ID NO: 15; and
   (3) an antibody or an antibody fragment thereof that comprises a heavy chain variable region comprising CDR1 having an amino acid sequence of SEQ ID NO: 16, CDR2 having an amino acid sequence of SEQ ID NO: 17, and CDR3 having an amino acid sequence of SEQ ID NO: 18, and a light chain variable region comprising CDR1 having an amino acid sequence of SEQ ID NO: 19, CDR2 having an amino acid sequence of SEQ ID NO: 20, and CDR3 having an amino acid sequence of SEQ ID NO: 21.
[10] An immunoassay kit for detecting SARS-CoV-2 in a biological sample, comprising two types of monoclonal antibodies or antibody fragments thereof that bind to a peptide fragment having 30 or less consecutive amino acids in a nucleocapsid protein of SARS-CoV-2, wherein the two types of monoclonal antibodies or antibody fragments thereof recognize different epitopes, and wherein the peptide fragment having 30 or less consecutive amino acids is a peptide fragment having an amino acid sequence represented by KQQTVTLLPAADLDDFSKQLQQSMSSA (SEQ ID NO: 1).
[11] The immunoassay kit according to [10], wherein the biological sample is a respiratory secretion.
[12] The immunoassay kit according to [10] or [11], wherein the two types of monoclonal antibodies or antibody fragments thereof are a first monoclonal antibody or an antibody fragment thereof and a second monoclonal antibody or an antibody fragment thereof, wherein the first monoclonal antibody or the antibody fragment thereof has a labeling substance indirectly or directly bound thereto, and the second monoclonal antibody or the antibody fragment thereof is indirectly or directly bound to a solid phase.
[13] The immunoassay kit according to [12], wherein the first monoclonal antibody or the antibody fragment thereof is a monoclonal antibody or an antibody fragment thereof that recognizes an epitope in the amino acid sequence represented by KQQTVTLLPAADLDFSK (SEQ ID NO: 2), and the second monoclonal antibody or the antibody fragment thereof is a monoclonal antibody or an antibody fragment thereof that recognizes an epitope in the amino acid sequence represented by AADLDDFSKQLQQSMSSA (SEQ ID NO: 3).
[14] The immunoassay kit according to any one of [10] to [12], wherein one of the monoclonal antibodies recognizes an epitope in the amino acid sequence represented by KQQTVTLLPAADLDDFSK (SEQ ID NO: 2), while the other monoclonal antibody recognizes an epitope in the amino acid sequence represented by AADLDDFSKQLQQSMSSA (SEQ ID NO: 3).
[15] The immunoassay kit according to any one of [10] to [13], wherein the two types of monoclonal antibodies or antibody fragments thereof are, respectively, a monoclonal antibody or an antibody fragment thereof that recognizes an amino acid sequence represented by LLPAA (SEQ ID NO: 26) as an epitope, and a monoclonal antibody or an antibody fragment thereof that recognizes an epitope in an amino acid sequence represented by LDDFSKQLQ (SEQ ID NO: 27).
[16] The immunoassay kit according to any one of [10] to [15], which is a kit for immunochromatography or ELISA.
[17] The immunoassay kit according to any one of [10] to [16], wherein the two types of monoclonal antibodies or the antibody fragments thereof are selected from the group consisting of:
   (1) an antibody or an antibody fragment thereof that comprises a heavy chain variable region comprising CDR1 having an amino acid sequence of SEQ ID NO: 4, CDR2 having an amino acid sequence of SEQ ID NO: 5, and CDR3 having an amino acid sequence of SEQ ID NO: 6, and a light chain variable region comprising CDR1 having an amino acid sequence of SEQ ID NO: 7, CDR2 having an amino acid sequence of SEQ ID NO: 8, and CDR3 having an amino acid sequence of SEQ ID NO: 9;
   (2) an antibody or an antibody fragment thereof that comprises a heavy chain variable region comprising CDR1 having an amino acid sequence of SEQ ID NO: 10, CDR2 having an amino acid sequence of SEQ ID NO: 11, and CDR3 having an amino acid sequence of SEQ ID NO: 12, and a light chain variable region comprising CDR1 having an amino acid sequence of SEQ ID NO: 13, CDR2 having an amino acid sequence of SEQ ID NO: 14, and CDR3 having an amino acid sequence of SEQ ID NO: 15; and
   (3) an antibody or an antibody fragment thereof that comprises a heavy chain variable region comprising CDR1 having an amino acid sequence of SEQ ID NO: 16, CDR2 having an amino acid sequence of SEQ ID NO: 17, and CDR3 having an amino acid sequence of SEQ ID NO: 18, and a light chain variable region comprising CDR1 having an amino acid sequence of SEQ ID NO: 19, CDR2 having an amino acid sequence of SEQ ID NO: 20, and CDR3 having an amino acid sequence of SEQ ID NO: 21.[Advantageous Effects of Invention]

The present invention can provide a SARS-CoV-2 immunoassay kit and a SARS-CoV-2 immunoassay method that enable highly sensitive and rapid immunoassay.

### [Brief Description of Drawings]

FIG. 1 is diagram showing the amino acid sequence of a nucleocapsid protein.
FIG. 2 is diagram showing the corresponding relationship between the amino acid sequence of a synthetic peptide used for epitope analysis for an antibody and the amino acid sequence of a nucleocapsid protein.

### [Description of Embodiments]

### Immunoassay method for assaying SARS-CoV-2

### (Biological sample)

Examples of the "biological sample" in the present specification include solid tissues and body fluids derived from living bodies.

More specific examples of biological samples include blood, serum, plasma, urine, tears, ear discharge, prostatic fluid, and respiratory secretions. Respiratory secretions are preferred. In the context of the present specification, the term "respiratory secretion" means bodily fluids secreted in or on the tissues of the respiratory organs. Examples of respiratory secretions include bodily fluids secreted in the nostrils, nasal cavities, pharynx, nasopharynx, oral cavity, trachea, bronchi, and lungs, and particularly preferred examples include nasopharyngeal swabs, nasal swabs, saliva, and sputum. In the context of the present specification, "respiratory organs" is a generic term for organs related to respiration, and covers organs ranging from the nasal vestibule to the alveoli (lungs) via the nasal cavity, pharynx, larynx, trachea, bronchi, and bronchioles.

Examples of subjects from which the biological sample is to be collected include humans or animals (e.g., monkeys, dogs, and cats), of which humans are preferable. The biological sample may be a biological sample as it is taken from a subject, or may be a sample obtained by subjecting a collected biological sample to treatments such as dilution and concentration that are usually performed. The person who collects and prepares a biological sample used in the immunoassay method of the present invention may or may not be identical to the person who performs the immunoassay method of the present invention. Further, the biological sample used in the present invention may be one collected or prepared during implementation of the immunoassay method of the present invention, or one previously collected or prepared and stored.

In the context of the present specification, the term "epitope" means a portion of an antigen (SARS-CoV-2 in the case of the present invention), which is recognized by an antibody.

### (SARS-CoV-2)

SARS-CoV-2 is a virus that causes the coronavirus disease 2019 (COVID-19). SARS-CoV-2 virus particles are composed of four proteins known as the spike protein, nucleocapsid protein, membrane protein, and envelope protein, as well as RNA.

The SARS-CoV-2 nucleocapsid protein is a protein composed of 419 amino acids (SEQ ID NO: 22). Mutations can occur in the nucleocapsid protein and therefore the nucleocapsid protein contains peptide fragments having amino acid sequences with at least 90 % identity (e.g., 91 %, 92 %, 93 %, 94 %, 95 %, 96 %, 97 %, 98 %, or 99 % identity) with the amino acid sequence represented by SEQ ID NO: 22.

The nucleocapsid protein contains an NTD antigen (containing the N-terminal RNA-binding domain), a CTD antigen (containing the C-terminal dimerization domain), and a Ser/Arg (SR)-rich linker domain located in the middle.

The NTD antigen is a region with amino acids 1 to 174 of the nucleocapsid protein. Among them, the region with the 1st to 49th amino acids is the N-terminal region, and the region with the 50th to 174th amino acids is the RNA binding domain.

The amino acid sequence of the NTD antigen is shown below.

MSDNGPQNQRNAPRITFGGPSDSTGSNQNGERSGARSKQRRPQGLPNNTASWF
TALTQHGKEDLKFPRGQGVPINTNSSPDDQIGYYRRATRRIRGGDGKMKDLSPR
WYFYYLGTGPEAGLPYGANKDGIIWVATEGALNTPKDHIGTRNPANNAAIVLQ
LPQGTTLPKGFYAE (SEQ ID NO: 23)

The linker domain is a region with amino acids 175 to 247 of the nucleocapsid protein.

The amino acid sequence of the linker domain is shown below.

GSRGGSQASSRSSSRSRNSSRNSTPGSSRGTSPARMAGNGGDAALALLLLDRLN

QLESKMSGKGQQQQGQTVT (SEQ ID NO: 24)

The CTD antigen is a region with amino acids 248 to 419 of the nucleocapsid protein. Among them, the region with the 248th to 364th amino acids is the dimerization domain, and the region with the 365th to 419th amino acids is the C-terminal region.

The amino acid sequence of the CTD antigen is shown below.

KKSAAEASKKPRQKRTATKAYNVTQAFGRRGPEQTQGNFGDQELIRQGTDYKH
WPQIAQFAPSASAFFGMSRIGMEVTPSGTWLTYTGAIKLDDKDPNFKDQVILLN
KHIDAYKTFPPTEPKKDKKKKADETQALPQRQKKQQTVTLLPAADLDDFSKQL
QQSMSSADSTQA (SEQ ID NO: 25)

### (Monoclonal antibody)

In the context of the present specification, the term "monoclonal antibody" refers to an antibody or antibody molecule that is obtained from clones derived from a single antibody-producing cell. In the immunoassay method of the present invention, an antibody fragment possessing the function of monoclonal antibody can also be used as long as the effects of the present invention can be achieved. Examples of antibody fragments possessing the function of monoclonal antibody include a functional fragment including the Fab portion of a monoclonal antibody obtained by enzymatic digestion of the monoclonal antibody, a functional fragment including the Fab portion of a monoclonal antibody produced by genetic recombination, a functional fragment including scFv produced by phage display method, and the like.

### (Peptide fragment having 30 or less consecutive amino acids)

The monoclonal antibody or the antibody fragment thereof used in the immunoassay method of the present invention binds to a peptide fragment having 30 or less consecutive amino acids in the nucleocapsid protein of SARS-CoV-2. Hereinafter, two types of monoclonal antibodies or antibody fragments thereof that bind to a peptide fragment having 30 or less consecutive amino acids in the nucleocapsid protein of SARS-CoV-2 may be referred to as "monoclonal antibody pair of the present invention". Further, one of the monoclonal antibodies constituting the monoclonal antibody pair of the present invention may be referred to as "monoclonal antibody of the present invention".

With respect to the monoclonal antibody pair of the present invention, it is preferable that both of the monoclonal antibodies bind to one peptide fragment having 27 to 30 consecutive amino acids in the nucleocapsid protein of SARS-CoV-2. According to the invention, both of the monoclonal antibodies bind to a peptide fragment having an amino acid sequence represented by KQQTVTLLPAADLDDFSKQLQQSMSSA (SEQ ID NO: 1) present in the C-terminal region of the nucleocapsid protein. Preferably, one of the monoclonal antibodies recognizes an epitope in the amino acid sequence represented by KQQTVTLLPAADLDDFSK (SEQ ID NO: 2), while the other monoclonal antibody recognizes an epitope in the amino acid sequence represented by AADLDDFSKQLQQSMSSA (SEQ ID NO: 3), and it is most preferable that one of the monoclonal antibodies recognizes the amino acid sequence represented by LLPAA (SEQ ID NO: 26) as an epitope, while the other monoclonal antibody recognizes an epitope in the amino acid sequence represented by LDDFSKQLQ (SEQ ID NO: 27).

The different antibodies of the monoclonal antibody pair of the invention recognize different epitopes. To recognize different epitopes in this context means that the amino acid sequences recognized as epitopes by the two monoclonal antibodies do not overlap.

Each of the antibodies of the monoclonal antibody pair of the invention may be an isolated monoclonal antibody or an antibody fragment thereof.

Using "two types" of monoclonal antibodies or antibody fragments thereof requires no more than using "at least two types" of the monoclonal antibodies of the present invention, and does not exclude embodiments using more than two types of the monoclonal antibodies of the present invention from the scope of the present invention.

In the present specification, a region with the 365th to 419th amino acids in the nucleocapsid protein of SARS-CoV-2 is referred to as "C-terminal region". According to the invention, each antibody of the monoclonal antibody pair of the present invention binds to a peptide fragment having 30 or less consecutive amino acids present in this C-terminal region. That is, the peptide fragment having 30 or less consecutive amino acids is located in the C-terminal region of the nucleocapsid protein of SARS-CoV-2. It has been reported that amino acid mutations of SARS-CoV-2 are less likely to occur in the C-terminal region. Therefore, antibodies that recognize the C-terminal region are more likely to be able to bind to SARS-CoV-2 with amino acid mutations than antibodies that recognize other regions.

Each antibody of the monoclonal antibody pair of the present invention preferably specifically recognizes an epitope present in a peptide fragment having 30 or less consecutive amino acids in the nucleocapsid protein of SARS-CoV-2. In this context, "specifically recognizes an epitope" means that the substantially no binding to anything other than a specific peptide fragment or epitope occurs.

In order to determine whether one of the antibodies of the monoclonal antibody pair of the present invention "does not substantially bind" to a peptide fragment having a certain amino acid sequence, for example, it is possible to perform a measurement based on the SPR method, using Biacore (registered trademark) T100 or T200, while having one of the antibodies of the monoclonal antibody pair of the present invention being immobilized. Whether the monoclonal antibody "does not substantially bind" can also be determined by other methods or means well known to those skilled in the art than the above SPR method.

The monoclonal antibody pair of the present invention is preferably a pair of two types of antibodies selected from the group consisting of (1) to (3) below.
(1) An antibody or an antibody fragment thereof that comprises a heavy chain variable region comprising CDR1 having an amino acid sequence of SEQ ID NO: 4, CDR2 having an amino acid sequence of SEQ ID NO: 5, and CDR3 having an amino acid sequence of SEQ ID NO: 6, and a light chain variable region comprising CDR1 having an amino acid sequence of SEQ ID NO: 7, CDR2 having an amino acid sequence of SEQ ID NO: 8, and CDR3 having an amino acid sequence of SEQ ID NO: 9.
(2) An antibody or an antibody fragment thereof that comprises a heavy chain variable region comprising CDR1 having an amino acid sequence of SEQ ID NO: 10, CDR2 having an amino acid sequence of SEQ ID NO: 11, and CDR3 having an amino acid sequence of SEQ ID NO: 12, and a light chain variable region comprising CDR1 having an amino acid sequence of SEQ ID NO: 13, CDR2 having an amino acid sequence of SEQ ID NO: 14, and CDR3 having an amino acid sequence of SEQ ID NO: 15.
(3) An antibody or an antibody fragment thereof that comprises a heavy chain variable region comprising CDR1 having an amino acid sequence of SEQ ID NO: 16, CDR2 having an amino acid sequence of SEQ ID NO: 17, and CDR3 having an amino acid sequence of SEQ ID NO: 18, and a light chain variable region comprising CDR1 having an amino acid sequence of SEQ ID NO: 19, CDR2 having an amino acid sequence of SEQ ID NO: 20, and CDR3 having an amino acid sequence of SEQ ID NO: 21.

In the above context, "CDR1 having an amino acid sequence of SEQ ID NO: 4" means that the CDR1 consists of an amino acid sequence of SEQ ID NO: 4. The same applies to other CDRs (complementarity-determining regions).

The monoclonal antibody of the present invention may include a constant region (C region) as well as the heavy chain variable region and the light chain variable region. As the constant region, a CL region may be included in the light chain, and a CH region (CH1, CH2, and CH3) may be included in the heavy chain.

The monoclonal antibody pair of the present invention is preferably a pair of two types of antibodies selected from the group consisting of (1) and (2) below.
(1) An antibody or an antibody fragment thereof that includes a heavy chain variable region comprising CDR1 having an amino acid sequence of SEQ ID NO: 4, CDR2 having an amino acid sequence of SEQ ID NO: 5, and CDR3 having an amino acid sequence of SEQ ID NO: 6, and a light chain variable region comprising CDR1 having an amino acid sequence of SEQ ID NO: 7, CDR2 having an amino acid sequence of SEQ ID NO: 8, and CDR3 having an amino acid sequence of SEQ ID NO: 9.
(2) An antibody or an antibody fragment thereof that includes a heavy chain variable region comprising CDR1 having an amino acid sequence of SEQ ID NO: 10, CDR2 having an amino acid sequence of SEQ ID NO: 11, and CDR3 having an amino acid sequence of SEQ ID NO: 12, and a light chain variable region comprising CDR1 having an amino acid sequence of SEQ ID NO: 13, CDR2 having an amino acid sequence of SEQ ID NO: 14, and CDR3 having an amino acid sequence of SEQ ID NO: 15.

The monoclonal antibody pair of the present invention is more preferably a pair of the antibody (1) and the antibody (2), and is most preferably a pair of the antibody (1) used as a labeled antibody and the antibody (2) used as a solid-phase antibody.

Examples of the antibody (1) include antibodies that recognize an epitope in the amino acid sequence represented by KQQTVTLLPAADLDFSK (SEQ ID NO: 2), such as S32213 antibody. Further examples of the antibody (1) include antibodies that recognize the amino acid sequence represented by LLPAA (SEQ ID NO: 26) as an epitope, such as S32213 antibody.

Examples of the antibody (2) include antibodies that recognize an epitope in the amino acid sequence of AADLDDFSKQLQQSMSSA (SEQ ID NO: 3), such as S32217 antibody. Further examples of the antibody (2) include antibodies that recognize an epitope in the amino acid sequence represented by LDDFSKQLQ (SEQ ID NO: 27), such as S32217 antibody.

Examples of the antibody (3) include antibodies that recognize an epitope in the CTD-side domain antigen, such as S32223 antibody. The S32223 antibody is presumed to have similar reactivity as the S32217 antibody.

The present specification includes descriptions indicating that a monoclonal antibody or an antibody fragment thereof "reacts with", "recognizes" or "binds to" a specific substance or amino acid sequence, which however are used interchangeably. Whether or not a monoclonal antibody "reacts with" an antigen (compound) can be determined through antigen-immobilized ELISA, competitive ELISA, sandwich ELISA, or the like. Alternatively, a method using the principle of surface plasmon resonance (SPR method) can also be used. The SPR method can be performed using equipment, sensors and reagents commercially available under the name Biacore (registered trademark).

For example, when the same procedure as the epitope analysis (antibody-epitope analysis 1) in Example 1 described below is followed, it can be appreciated that the amino acid sequence contained in the peptide fragment with the highest absorbance has been recognized as an epitope.

### (Method for preparing monoclonal antibody)

The monoclonal antibody of the present invention can be prepared by dissolving a full-length nucleocapsid protein as an antigen (immunogen) in a solvent such as phosphate-buffered saline, and administering the resulting solution to a non-human animal for immunization. Immunization may be performed using an emulsion after addition of an appropriate adjuvant to the solution as necessary. Examples of adjuvants include generally used adjuvants, such as water-in-oil emulsions, water-in-oil-in-water emulsions, oil-in-water emulsions, liposomes, and aluminum hydroxide gels, as well as proteins or peptide substances derived from biological components. For example, Freund's incomplete adjuvant or Freund's complete adjuvant can be suitably used. The administration route, dosage, and administration time for the adjuvant are not particularly limited, but are preferably selected appropriately so as to enhance the desired immune response in the animal to be immunized with the antigen.

The type of animal used for immunization is also not particularly limited, and mammals such as mice, rats, cows, rabbits, goats, sheep, or alpacas are preferred, and mice or rats are more preferred. Animals can be immunized following common techniques, for example, by subcutaneously, intracutaneously, intravenously, or intraperitoneally injecting an antigen solution, preferably a mixture thereof with an adjuvant, into the animals. Since the immune response generally differs depending on the type and strain of the animal to be immunized, it is desirable to appropriately set the immunization schedule according to the animal used. It is preferable to repeat the antigen administration several times after the initial immunization.

For obtaining the monoclonal antibody of the present invention, the following operations may be subsequently performed, which, however, are not essential and do not limit the present invention. Methods for producing monoclonal antibodies per se are well known and widely used in the art, and those skilled in the art can prepare the monoclonal antibody of the present invention by using the antigens as described above (see, for example, Antibodies, A Laboratory Manual (Cold Spring Harbor Laboratory Press, (1988), Chapter 6, etc.).

After the final immunization, antibody-producing spleen cells or lymph node cells are extracted from the immunized animal and fused with a myeloma-derived cell line having high proliferative potential to prepare hybridomas. Cells with high antibody-producing ability (in terms of quality and quantity) are preferably used for cell fusion, and it is more preferable that the myeloma-derived cell line is compatible with the animal from which the antibody-producing cells to be fused are derived. Cell fusion can be performed according to methods known in the art. For example, a polyethylene glycol method, a method using Sendai virus, a method using electric current, or the like can be employed. The resulting hybridomas can be grown according to conditions commonly used in the art. A desired hybridoma can be selected while checking the properties of the antibody to be produced. Hybridoma cloning can be performed by well-known methods such as the limiting dilution method and the soft agar method.

After the cloning step, the ability of the produced monoclonal antibody to bind to the full-length nucleocapsid protein can be assayed by methods such as ELISA, RIA, and immunofluorescence. These operations allow determination of whether the selected hybridomas produce monoclonal antibodies with the desired properties.

By mass-culturing the hybridomas selected as described above, monoclonal antibodies having the desired properties can be produced. The method for mass-culturing is not particularly limited, and examples thereof include a method that cultures hybridomas in an appropriate medium to produce monoclonal antibodies in the medium, and a method that injects hybridomas into the peritoneal cavity of a mammal to allow the hybridomas to proliferate, thereby producing monoclonal antibodies in ascites.

As the monoclonal antibody of the present invention, it is possible to use an antibody fragment of a monoclonal antibody having antigen-antibody reactivity as well as the whole antibody molecule. In addition to those obtained through the process of immunizing animals as described above, it is also possible to use monoclonal antibodies obtained using gene recombination techniques, such as chimeric antibodies, humanized antibodies and human antibodies. Examples of fragments of monoclonal antibodies include F(ab')₂, Fab', scFv, and the like. These fragments can be prepared by treating the monoclonal antibody obtained as described above with a protease (e.g., pepsin or papain), or cloning the DNA of the antibody and expressing it in a culture system using E. coli or yeast.

By using the monoclonal antibody pair of the present invention, a sandwich system can be constructed in the immunoassay method of the present invention. A sandwich system is a method that achieves high specificity and sensitivity by sandwiching a detection target substance between two types of antibodies that recognize different epitopes.

It is assumed that the use of two types of monoclonal antibodies or antibody fragments thereof that bind to a peptide fragment having 30 or less consecutive amino acids reduces the influence caused by amino acid mutations and peptide cleavage by enzymes, etc., resulting in higher sensitivity.

When constructing a sandwich system, it is preferable that at least one of the two types of monoclonal antibodies of the antibody pair of the present invention is a solid-phase antibody, and at least one is a labeled antibody. In the context of the present specification, the "solid-phase antibody" means a monoclonal antibody directly or indirectly immobilized on a solid phase. In the context of the present specification, the "labeled antibody" means a monoclonal antibody that is directly or indirectly labeled with a conventional labeling substance well known in the art described below when measuring a signal attributable to the labeling substance.

For example, a solid-phase antibody can be produced by allowing a monoclonal antibody to physically adsorb or chemically bind to a solid phase (optionally via an appropriate spacer). The solid phase to be used may be a solid phase composed of a polymer substrate such as polystyrene resin, an inorganic substrate such as glass, a polysaccharide substrate such as cellulose or agarose, or the like. The shape of the solid phase is not particularly limited, and any appropriate shape may be chosen, for example, from a plate shape (e.g., microplate or membrane), a bead or particulate shape (e.g., latex particles, magnetic particles), or a cylindrical shape (e.g., test tube).

The use of a labeled antibody (secondary antibody) capable of directly binding to the monoclonal antibody of the present invention also enables the measurement of amount of the antibody bound to SARS-CoV-2 or a peptide fragment thereof. In the present specification, an antibody which is bound to the monoclonal antibody of the present invention and has a labeling substance bound thereto is referred to as a "secondary antibody". This secondary antibody may be used to indirectly bind the labeling substance to the monoclonal antibody of the present invention.

By measuring the intensity of the signal generated by the labeling substance, the amount of SARS-CoV-2 or a peptide fragment thereof in the biological sample can be measured. Examples of labeling substances for preparing the labeled antibody include metal complexes, enzymes, insoluble particles, fluorescent substances, chemiluminescent substances, biotin, avidin, radioactive isotopes, colloidal gold particles, and colored latex. The method used for binding the labeling substance to the monoclonal antibody may be physical adsorption, glutaraldehyde method, maleimide method, pyridyl disulfide method, or periodic acid method, which are available to those skilled in the art. When an enzyme such as horseradish peroxidase (HRP) or alkaline phosphatase (ALP) is used as a labeling substance, enzymatic activity can be measured using a specific substrate for the enzyme. For example, as the substrate for measuring the enzymatic activity, o-phenylenediamine (OPD) or 3,3',5,5'-tetramethylbenzidine (TMB) can be used when the enzyme is HRP, and p-nitrophenyl phosphate can be used when the enzyme is ALP. When biotin is used as a labeling substance, a monoclonal antibody may be labeled with biotin and reacted with avidin or streptavidin labeled with an enzyme, dye, or fluorescent label (preferably HRP). In the immunoassay method of the present invention, it is preferable to use colloidal gold particles as the labeling substance.

In the context of the present specification, the process of physically or chemically supporting an antigen or antibody on a solid phase or the state of an antigen or antibody being supported on a solid phase is sometimes referred to as "immobilization" or " solid-phase immobilization". The term "assay", "detection" or "measurement" also encompasses proving the presence of SARS-CoV-2 or a peptide fragment thereof and quantification of SARS-CoV-2 or a peptide fragment thereof.

The immunoassay method of the present invention uses two types of monoclonal antibodies that recognize different epitopes. For convenience, one of the monoclonal antibodies may be referred to as "first monoclonal antibody", while the other one of the monoclonal antibodies may be referred to as "second monoclonal antibody".

When the first monoclonal antibody is a labeled antibody and the second monoclonal antibody is a solid-phase antibody, the immunoassay method of the present invention may include the following steps (1) to (3).
(1) A step of contacting a biological sample with a first monoclonal antibody having a labeling substance bound thereto to form a first complex (containing SARS-CoV-2 or a peptide fragment thereof, the labeling substance, and the first monoclonal antibody).
(2) A step of contacting the first complex with a second monoclonal antibody to form a second complex (containing SARS-CoV-2 or a peptide fragment thereof, the labeling substance, the first monoclonal antibody, and the second monoclonal antibody).
(3) A step of measuring a signal attributable to the labeling substance.

The second complex contains a labeling substance. For signal measurement, a measurement method well known in the art can be employed depending on the labeling substance. The signal measurement may be performed using a measuring instrument, or may be performed visually.

The immunoassay method of the present invention may include, if necessary, a step of pretreating the biological sample and/or a step of comparing the intensity of the obtained signal with a first threshold. Examples of pretreatments include filtration of the biological sample and dilution of the biological sample with a sample diluent. The first threshold may be appropriately set in consideration of sensitivity and type of the biological sample, etc.

The first threshold may be a numerical range. The first threshold being a numerical range means that the specified range include a specific threshold, and the determination of whether the measured value is larger or smaller than the specific threshold allows determination of the presence or absence of a disease.

The first threshold can be, for example, 1.1 × 10²TCID₅₀/mL.

When the first threshold is a numerical range, the first threshold may be in a range between 1.1 × 10²TCID₅₀/mL and 2.0 × 10²TCID₅₀/mL, a range between 1.1 × 10²TCID₅₀/mL and 1.8 × 10²TCID₅₀/mL, or a range between 1.1 × 10²TCID₅₀/mL to 1.5 × 10² TCID₅₀/mL.

The immunoassay method may include a step of determining that the subject is infected with SARS-CoV-2 when the signal intensity is lower (higher) than the first threshold, or a step of determining that the subject is not infected with SARS-CoV-2 when the signal intensity is higher (lower) than the first threshold.

In a further aspect falling outside the scope of the claims the immunoassay method can determine the therapeutic efficacy of a specific drug in a subject infected with SARS-CoV-2 based on the measured signal values. In this instance, the immunoassay method may further include the following step in addition to the steps described above.

A step of administering a specific drug to a subject and/or comparing the intensity of the signal with a second threshold.

In this instance, the second threshold can be appropriately set in consideration of the sensitivity and the type of biological sample, but may be the measured value of SARS-CoV-2 or a peptide fragment thereof in the subject prior to administration of a specific drug.

The immunoassay method may include a step of determining that a specific drug has a therapeutic efficacy when the signal intensity is lower (higher) than the second threshold, or a step of determining that a specific drug has no therapeutic efficacy when the signal intensity is higher (lower) than the second threshold.

In the determination of the therapeutic efficacy, the therapeutic efficacy may be monitored by conducting measurement every few days.

### (Immunoassay method)

The "immunoassay method" is a method of measuring the level of a substance contained in a biological sample using the reaction between an antigen and an antibody. The term "level" encompasses the amount, concentration, or determination of presence or absence of a substance.

Examples of the immunoassay method of the present invention include, but not limited to, electrochemiluminescence immunoassay (ECL method), enzyme-linked immunosorbent assay (ELISA), latex immunoturbidimetric assay (LTIA method), chemiluminescence immunoassay, immunochromatography, and immunofluorescence. The immunoassay method of the present invention is preferably ELISA or immunochromatography, more preferably immunochromatography.

The immunoassay method of the present invention is an in vitro immunoassay method. Further, a sensitizer may also be used to enhance sensitivity.

The immunoassay method can assay SARS-CoV-2 or a peptide fragment thereof contained in a biological sample in an amount equivalent to 1.1 x 10²TCID₅₀/mL or more. In the immunoassay method of the present invention, the order of adding the monoclonal antibody of the present invention and the biological sample to the measurement system is not limited as long as the effects of the present invention can be achieved. That is, the monoclonal antibody of the present invention may be added to the measurement system before the addition of the biological sample, or simultaneously with the addition of the biological sample, or after the addition of the biological sample.

The measurement procedure and principle are described below for each immunoassay method to be employed. The following descriptions are presented for illustrative purpose only with respect to the measurement procedure and principle for one embodiment of the present invention, and by no means limit the scope of the present invention.

In the immunoassay method of the present invention, it is preferable to use a monoclonal antibody or an antibody fragment thereof that recognizes an epitope in the amino acid sequence represented by KQQTVTLLPAADLDDFSK (SEQ ID NO: 2) as a labeled antibody, while using a monoclonal antibody or an antibody fragment thereof that recognizes an epitope in the amino acid sequence represented by AADLDDFSKQLQQSMSSA (SEQ ID NO: 3) as a solid-phase antibody, and it is more preferable to use a monoclonal antibody or an antibody fragment thereof that recognizes, as an epitope, the amino acid sequence represented by LLPAA (SEQ ID NO: 26) as a labeled antibody, while using a monoclonal antibody or an antibody fragment thereof that recognizes an epitope in the amino acid sequence represented by LDDFSKQLQ (SEQ ID NO: 27) as a solid-phase antibody.

In each of the immunoassay methods described below, methods known in the art including those described above can be used without any limitation with respect to specific methods such as the method for immobilizing the monoclonal antibody on the solid phase, the method for binding the monoclonal antibody to the labeling substance, and the type of labeling substance.

### (Immunochromatography)

Immunochromatography is an immunoassay method that utilizes the behavior that a labeled antibody bound to a target substance to be detected or a labeled antibody flows on a membrane. A general principle in measuring a target substance to be detected by the immunochromatography is as follows.

An antibody against an antigen (target substance to be detected) is immobilized on an insoluble membrane carrier, which is a chromatographic medium, to prepare a detector unit, which is a stationary phase. Then, a conjugate (labeling substance sensitized by an antibody capable of binding to the target substance to be detected) is used as a mobile phase. The target substance to be detected and the conjugate as a mobile phase are allowed to react specifically, and the resulting the target substance bound to the conjugate is allowed to react, in the detector unit as a stationary phase, specifically with the antibody immobilized on the detector unit.

The measurement procedure and principle when the immunochromatography is employed as the immunoassay method of the present invention are as follows.
(1) A biological sample is brought into contact with a sample supply unit for supplying the biological sample.
(2) SARS-CoV-2 or a peptide fragment thereof in the biological sample is allowed to contact the conjugate, thereby forming a first complex (including SARS-CoV-2 or a peptide fragment thereof, the labeling substance, and the first monoclonal antibody). The conjugate is a labeling substance having the first monoclonal antibody bound thereto.
(3) The first complex and the second monoclonal antibody immobilized on the detector unit come into contact, thereby forming a second complex (including SARS-CoV-2 or a peptide fragment thereof, the labeling substance, the first monoclonal antibody, and the second monoclonal antibody).
(4) The intensity of the signal attributable to the labeling substance contained in the conjugate is measured to confirm the formation of the second complex.

Examples of labeling substances include colloidal gold particles, colloidal platinum particles, color latex particles, magnetic particles, and the like. Among these, colloidal gold particles are preferable. Those skilled in the art can appropriately select and adjust the type and particle size of these labeling substances according to the desired sensitivity.

### (ELISA)

Among various immunoassay methods, ELISA using an enzyme label is also preferable because the target can be measured easily and quickly. In the context of the present specification, the "ELISA" means a method in which an antigen or antibody as a target substance to be detected contained in a sample is trapped with an antibody or antigen corresponding to the target substance, and then detected using an enzymatic reaction. The solid phase is preferably a plate (immunoplate). HRP or ALP can be used as a label.

The measurement procedure and principle when the sandwich ELISA is used as the immunoassay method of the present invention are as follows.
(1) When a biological sample is added to a solid phase on which a solid-phase antibody is immobilized and allowed to react, SARS-CoV-2 or a peptide fragment thereof in the biological sample binds to the solid-phase antibody to form a complex of the solid-phase antibody and the SARS-CoV-2 or the peptide fragment thereof on the solid phase.
(2) When a labeled antibody that recognizes another labeled epitope is added to the solid phase and allowed to react, the labeled antibody binds to the trapped SARS-CoV-2 or peptide fragment thereof and forms a sandwich with the above complex of the solid-phase antibody and the SARS-CoV-2 or the peptide fragment thereof.
(3) After washing, the resulting is reacted with an enzyme substrate to develop color, and the absorbance is measured.

The amount of SARS-CoV-2 or a peptide fragment thereof in the biological sample can be measured according to the amount of labeling substance measured.

A secondary antibody can also be used in the sandwich ELISA. The use of a secondary antibody can amplify the reaction and enhance the detection sensitivity. In the case of an example given below, the secondary antibody is an antibody that specifically recognizes the primary antibody (second monoclonal antibody).

When using a secondary antibody, the following procedures (1) to (5) can be adopted.
(1) A biological sample that has been appropriately treated and diluted is added to a solid phase with a first monoclonal antibody immobilized thereon, followed by incubation, removal of the biological sample and washing.
(2) The primary antibody (second monoclonal antibody) is added, followed by incubation and washing.
(3) Further, an enzyme-labeled secondary antibody is added and incubated.
(4) A substrate is added for color development.
(5) The amount of SARS-CoV-2 or peptide fragments thereof is assayed by measuring color development using a plate reader or the like.

### (Electrochemiluminescence immunoassay)

Electrochemiluminescence immunoassay means a method in which a labeling substance is caused to emit light by application of electric current, and the amount of light emitted is detected to measure the amount of a target substance to be detected. A ruthenium complex can be used as a labeling substance in the electrochemiluminescence immunoassay. An electrode is placed on a solid phase (such as a microplate), and radicals are generated on the electrode to excite the ruthenium complex to emit light. Then, the amount of light emitted from this ruthenium complex can be detected.

The measurement procedure and principle when using the magnetic particles as the solid phase, and the ruthenium complex as the labeling substance are as follows.
(1) When the magnetic particles on which the solid-phase antibody is immobilized are brought into contact with the biological sample, SARS-CoV-2 or a peptide fragment thereof in the biological sample binds to the solid-phase antibody.
(2) When the labeled antibody is brought into contact with the magnetic particles after washing, the labeled antibody binds to the SARS-CoV-2 or the peptide fragment thereof bound to the magnetic particles.
(3) After the magnetic particles are washed, the application of electric current causes light emission depending on the amount of the labeled antibody bound to the SARS-CoV-2 or the peptide fragment thereof. By measuring the amount of luminescence, the amount of the target substance to be detected in the biological sample can be accurately measured.

### (Latex immunoturbidimetry)

Latex immunoturbidimetry is an immunoassay method that utilizes agglutination of an antibody bound to the surface of latex and a target substance to be detected (antigen). The latex particles are not particularly limited as long as they are latex particles generally used for in vitro diagnostic agents. With respect to the latex particles during the agglutination reaction measurement, the concentration, average particle size and the like can be appropriately set according to the sensitivity or performance.

The measurement procedure and principle when the latex immunoturbidimetry is used as the immunoassay method of the present invention are as follows.
(1) A first monoclonal antibody and a second monoclonal antibody are bound to latex particles and brought into contact with a biological sample.
(2) SARS-CoV-2 or a peptide fragment thereof in the biological sample binds to the first monoclonal antibody and the second monoclonal antibody, resulting in agglutination of the antibody-bound latex particles.
(3) The biological sample is irradiated with near-infrared light to measure absorbance or scattered light. Based on the measurements, the antigen concentration can be determined.

When the latex immunoturbidimetry is used as the immunoassay method of the present invention, the latex is a solid phase and acts as a labeling substance as well. That is, both the first monoclonal antibody and the second monoclonal antibody are bound to each of the solid phase and the labeling substance.

### 2. Immunoassay kit for assaying SARS-CoV-2 in biological sample

The immunoassay kit of the present invention for assaying SARS-CoV-2 in a biological sample (hereinafter, also referred to simply as "immunoassay kit of the present invention") includes the monoclonal antibody pair of the present invention. The different antibodies of the monoclonal antibody pair of the invention recognize different epitopes. The different antibodies of the monoclonal antibody pair of the present invention may be placed in separate containers.

Examples of the immunoassay kit of the present invention include, but not limited to, immunoassay kits for performing immunochromatography, ELISA, electrochemiluminescence immunoassay, latex immunoturbidimetry, chemiluminescence immunoassay, and immunofluorescence. The immunoassay kit of the present invention is preferably an immunoassay kit for performing ELISA or immunochromatography, and more preferably an immunoassay kit for performing immunochromatography.

The immunoassay kit of the present invention can be an immunoassay kit for assaying in vivo or in vitro samples.

The immunoassay kit of the present invention can also include other test reagents such as standard antigen substances and quality control antigen samples, specimen diluents, and/or an instruction manual, etc. A person skilled in the art can appropriately adjust the concentrations of the antibody-containing reagent and the like.

The reagents contained in the kit are described below for each immunoassay method to be employed. In the immunoassay kit of the present invention, it is preferable to use a monoclonal antibody or an antibody fragment thereof that recognizes an epitope in the amino acid sequence represented by KQQTVTLLPAADLDDFSK (SEQ ID NO: 2) as a labeled antibody, while using a monoclonal antibody or an antibody fragment thereof that recognizes an epitope in the amino acid sequence represented by AADLDDFSKQLQQSMSSA (SEQ ID NO: 3) as a solid-phase antibody, and it is more preferable to use a monoclonal antibody or an antibody fragment thereof that recognizes, as an epitope, the amino acid sequence represented by LLPAA (SEQ ID NO: 26) as a labeled antibody, while using a monoclonal antibody or an antibody fragment thereof that recognizes an epitope in the amino acid sequence represented by LDDFSKQLQ (SEQ ID NO: 27) as a solid-phase antibody.

When using immunochromatography, the immunoassay kit of the present invention may take a form in which an immunochromatography test strip is stored and mounted in a suitable container (housing).

The immunochromatography test strip may be composed of a sample pad having a sample supply unit, an insoluble membrane carrier as a chromatography medium, and an absorbent pad disposed at the downstream end of the insoluble membrane carrier.

A detection unit with a first monoclonal antibody immobilized thereon may be placed on the insoluble membrane carrier, and a conjugate pad with a conjugate placed thereon may be placed between the sample pad and the insoluble membrane carrier.

The conjugate may be placed on the sample pad or the insoluble membrane carrier.

As regards other configurations of immunochromatography, for example, those described in International Publication No. 2018/012517 or International Publication No. 2016/031892 may be appropriately adopted.

Examples of labeling substances include colloidal gold particles, colloidal platinum particles, color latex particles, magnetic particles, and the like. Among these, colloidal gold particles are preferable. Those skilled in the art can appropriately adjust the types and particle sizes of these labeling substances.

When using sandwich ELISA, the immunoassay kit of the present invention may include the following (A) and (B): (A) a labeling reagent including a conjugate of a second monoclonal antibody and an enzyme (HRP, ALP, etc.); and (B) a solid phase having a first monoclonal antibody immobilized thereon.

In such a kit, first, a biological sample is added to a solid phase with a first monoclonal antibody immobilized thereon, followed by incubation, removal of the biological sample, and washing. Next, a labeling reagent is added, followed by incubation and addition of a substrate for color development. Thereafter, SARS-CoV-2 or peptide fragments thereof can be assayed by measuring color development using a plate reader or the like.

When using the electrochemiluminescence immunoassay, the immunoassay kit of the present invention may include the following (A) and (B).
(A) A labeling reagent including a conjugate of a second monoclonal antibody and an electrochemiluminescent substance (e.g., a ruthenium complex, etc.).
(B) A solid phase having immobilized thereon a first monoclonal antibody that binds to SARS-CoV-2 or a peptide fragment thereof.

For example, in a kit using magnetic particles as the solid phase, the biological sample is added to the magnetic particles on which the first monoclonal antibody that binds to SARS-CoV-2 or a peptide fragment thereof is immobilized and allowed to react, followed by removing the biological sample and washing. The conjugate is then added and allowed to react. After washing the magnetic particles, electrical energy is applied to cause light emission. Thereafter, SARS-CoV-2 or a peptide fragment thereof can be assayed by measuring the amount of light emitted from the labeling substance.

When using the latex immunoturbidimetry, the immunoassay kit of the present invention may include the following (1) and (2).
(1) Latex particles having a first monoclonal antibody bound thereto.
(2) Latex particles having a second monoclonal antibody bound thereto.

When the immunoassay kit of the present invention is a kit for latex immunoturbidimetry, the latex serves as a solid phase and a labeling substance as well. Thus, both the first monoclonal antibody and the second monoclonal antibody are bound to each of the solid phase and the labeling substance.

Thus, the descriptions are given above separately for various aspects of the invention, but the descriptions, definitions of terms, and embodiments described for a particular aspect are also applicable to the other aspects.

Hereinbelow, the present invention will be described in detail with reference to examples, which however should not be construed as limiting the present invention. In the following description, the unit "%" refers to "% by mass", unless otherwise specified.

### [Examples]

### [Preparation Example 1 Preparation of monoclonal antibody]

SARS-CoV-2 (COVID-19) nucleocapsid protein, His-tag 1-419 aa (NUN-C5227 manufactured by ACROBiosystems) (hereinafter referred to as "Nu antigen") was used as an immunogen. The Nu antigen was mixed at 1:1 with Freund's Complete Adjuvant (Difco Laboratories) for the first immunization and with Freund's Complete Adjuvant (Difco Laboratories) for the second and subsequent immunizations. For Balb/c, subcutaneous immunization was continuously carried out every other week with an immunogen amount of 20 µg for the initial round of immunization, and 10 µg (diluted with PBS) for the second and subsequent rounds of immunization. The blood antibody titers were evaluated by antigen-immobilized ELISA after implementing the third round of immunization. Individuals showing a sufficient increase in titer were intraperitoneally immunized with an immunogen diluted with PBS one to three days before dissection. Then, spleen cells, iliac lymph node cells and inguinal lymph node cells were collected and fused with myeloma cell SP2/0 by electrofusion method. The fused cells were cultured on a 96-well plate, and the culture supernatant was collected 7 or 8 days after the fusion. Thereafter, screening was performed by antigen-immobilized ELISA described below, and strains showing reactivity to the Nu antigen but not to NHis-cBSA were selected. In this process, the medium was exchanged on the day before the screening.

### [Preparation Example 2 Screening of anti-Nu antigen antibody (antigen-immobilized ELISA)]

The Nu antigen and NHis-cBSA (His-tag antigen conjugated to BSA (in-house preparation), 100 ng/mL in PBS) were dispensed into a 96-well plate for ELISA (NUNC442404) (50 µL/well) and allowed to stand at room temperature for 2 hours. After washing 3 times with PBST, a blocking solution (1% BSA-PBST) was dispensed (100 µL/well) and the resulting was allowed to stand at room temperature for 1 hour or at 4 °C overnight. After removing the blocking solution, a cell culture supernatant (2-fold diluted) and an antiserum (1000- and 10000-fold diluted) were dispensed (50 µL/well) and the resulting was allowed to stand at room temperature for 1 hour. After washing 3 times with PBST, Goat anti-Mouse IgG (H+L) PAb-HRP (1031-05 manufactured by Southern Biotech, 9500-fold diluted) was dispensed (50 µL/well) and the resulting was allowed to stand at room temperature for 1 hour. After washing 3 times with PBST, an OPD coloring solution was dispensed (50 µL/well) and the resulting was allowed to stand at room temperature for 10 minutes. A stop solution was dispensed (50 µL/well), and after stopping the reaction, measurement was performed using a plate reader (Abs. 492 nm). Antibodies that showed reactivity to the Nu antigen but showed no reactivity to the NHis-cBSA were selected. Based on the reactivity, etc., the selected antibodies were classified into Group A (3 types), Group B (15 types), Group C1 (8 types), Group C2 (3 types), Group C3 (3 types), Group C4 (1 type), and Group D (1 type).

### [Assay Example 1 Antibody classification by sandwich ELISA]

The following procedure was followed to investigate whether the Nu antigen could be detected by performing sandwich ELISA using two types of antibodies selected from the obtained antibodies.

One type of the antibody was dispensed into a 96-well plate for ELISA (NUNC442404) and allowed to stand at room temperature for 2 hours. After washing 3 times with PBST, a blocking solution (1% BSA-PBST) was dispensed (100 µL/well) and the resulting was allowed to stand at 4 °C overnight. After removing the blocking solution, the Nu antigen was dispensed and the resulting was allowed to stand at room temperature for 30 minutes. After washing 3 times with PBST, another type of the antibody labeled with biotin was added and the resulting was allowed to stand at room temperature for 1 hour. After washing 3 times with PBST, 50 µL/well of streptavidin-HRP diluted 5,000 times was dispensed and the resulting was allowed to stand at room temperature for 30 minutes. After washing 3 times with PBST, an OPD coloring solution was dispensed and the resulting was allowed to stand at room temperature for 10 minutes. A reaction stop solution was dispensed, and the absorbance was measured using a plate reader (wavelength: 492 nm).

When the Nu antigen could not be detected by sandwich ELISA, the antigen recognition sites of the two types of antibodies were judged to be close, and the two types of antibodies were classified into the same group. Table 1 below shows whether or not antibody pairs in each group could be sandwiched.

**[Table 1]**

| | | Solid-phase antibody | | | | | | |
|---|---|---|---|---|---|---|---|---|
| | | Group A | Group B | Group C1 | Group C2 | Group C3 | Group C4 | Group D |
| | Group A | - | + | + | + | + | + | + |
| Liquid-phase antibody | Group B | + | - | + | + | + | + | + |
| | Group C1 | + | + | - | - | + | - | + |
| | Group C2 | - | + | - | - | + | + | + |
| | Group C3 | + | + | + | + | - | - | + |
| | Group C4 | - | + | - | + | - | - | + |
| | Group D | + | - | - | - | - | + | - |

| | | | | | | | | |
|---|---|---|---|---|---|---|---|---|
| + : Sandwichable, - : Not sandwichable | | | | | | | | |

### [Assay Example 2 Antibody classification by immunochromatography]

An immunochromatography test strip was prepared by the following procedure.

### 1) Preparation of colloidal gold-labeled antibody solution

1 mL of phosphate buffer containing 25 µg/mL of anti-SARS-CoV-2 antibody was added to 20 mL of 1 OD/mL-colloidal gold solution, and the resulting mixture was stirred at room temperature for 10 minutes. Subsequently, 2 mL of 10 % BSA solution was added to the colloidal gold solution and the resulting was stirred at room temperature for 5 minutes. The obtained solution was centrifuged at 10,000 rpm for 45 minutes at 10 °C, and the supernatant was removed. The residue was suspended in Conjugate Dilution Buffer (Scripps) to obtain a gold colloid-labeled antibody solution.

### 2) Preparation of conjugate pad

The colloidal gold-labeled antibody solution prepared in 1) was diluted to 4 OD/mL with a 1.33 % casein, 4 % sucrose solution (pH 7.5) to prepare a conjugate solution. The conjugate solution was applied in lines onto a glass fiber pad and dried to obtain a conjugate pad.

### 3) Preparation of antibody-immobilized membrane

PBS containing 0.75 mg/mL anti-SARS-CoV-2 antibody and 2.5 % sucrose was prepared and used as a test line coating solution. PBS containing 1.0 mg/mL goat anti-mouse IgG monoclonal antibody and 2.5 % sucrose was prepared and used as a control line coating solution. Using a dispenser for immunochromatography "XYZ3050" (manufactured by BioDot), the test line coating solution and the control line coating solution were each applied on a nitrocellulose membrane at 1.0 µL/cm and dried to obtain an antibody-immobilized membrane.

### 4) Fabrication of test device

The antibody-immobilized membrane, the conjugate pad, and an absorbent pad were attached to a plastic adhesive sheet, and the resultant was cut into a 5 mm-width strip to obtain an immunochromatography test strip.

### Test Method

### 1) Sample

SARS-CoV-2 (Isolate: USA-WA1/2020) Culture Fluid (Heat Inactivated) (ZeptoMetrix) was diluted to 5.0 x 10⁵TCID₅₀/mL with Universal Transport Medium (BD). The resulting was further 11-fold diluted with a sample diluent for rapid tester FLU/NEXT (Sekisui Medical Co., Ltd.).

### 2) Test procedure

120 µL of the sample was dropped onto the test strip, and 10 minutes later, it was visually determined whether the test line on the antibody-immobilized membrane had developed color.

The results of detection tests for inactivated SARS-CoV-2 antigens using the test strips prepared with various antibody combinations are as shown in Table 2. It was found that the immunochromatography was able to detect the inactivated SARS-CoV-2 antigen in some antibody group pairs that could be sandwiched by ELISA.

**[Table 2]**

| | | Solid-phase antibody | | | | | | |
|---|---|---|---|---|---|---|---|---|
| | | Group A | Group B | Group C1 | Group C2 | Group C3 | Group C4 | Group D |
| | Group A | - | - | - | - | - | - | - |
| Labeled antibody | Group B | - | - | - | - | - | - | - |
| | Group C1 | - | - | - | - | + | - | - |
| | Group C2 | - | + | - | - | ++ | ++ | - |
| | Group C3 | - | - | - | + | - | - | - |
| | Group C4 | - | - | - | + | - | - | - |
| | Group D | - | - | - | - | - | + | - |

| | | | | | | | | |
|---|---|---|---|---|---|---|---|---|
| ++ : Sandwichable plus high sensitivity, + : Sandwichable, - : Not sandwichable | | | | | | | | |

### [Example 1 Antibody-epitope analysis 1]

From each of the antibody groups, one type of antibody was selected, and it was investigated whether the selected antibodies react with the N-terminal side or the C-terminal side of the Nu antigen.

An anti-His-tag antibody adjusted to 5 µg/mL was dispensed at 50 µL/well into a 96-well ELISA microplate and allowed to stand at 4 °C overnight. After washing 3 times with PBST, 100 µL/well of PBST (blocking solution) containing 1% BSA was dispensed and the resulting was allowed to stand at 4 °C overnight. After removing the blocking solution, the Nu antigen prepared to 100 ng/ml, SARS-CoV-2 (COVID-19) NP NTD domain V2 Recombinant Protein His-tag, 44-180 aa (hereinafter referred to as "NTD-side domain antigen", ProSci 92 -749), or Recombinant nucleoprotein (C-term) antigen for COVID-19 (NP-CTD), His-tag 212-417 aa (hereinafter referred to as "CTD-side domain antigen", rekom biotech, RAG0071) was dispensed at 50 µL/ml, and the resulting was allowed to stand at room temperature for 1 hour. After washing 3 times with PBST, each biotin-labeled antibody adjusted to 1 µg/ml was dispensed at 50 µL/well and the resulting was allowed to stand at room temperature for 1 hour. After washing 3 times with PBST, 50 µL/well of streptavidin-HRP diluted 5,000 times was dispensed and the resulting was allowed to stand at room temperature for 30 minutes. After washing 3 times with PBST, an OPD coloring solution was dispensed and the resulting was allowed to stand at room temperature for 10 minutes. A reaction stop solution was dispensed, and the absorbance was measured using a plate reader (wavelength: 492 nm).

The results are shown in Table 3 below.

**[Table 3]**

| Group | Antibody | Nu antigen | NTD-side domain antigen | CTD-side domain antigen |
|---|---|---|---|---|
| A | S32201 | 0.680 | 4.177 | 0.012 |
| B | S32202 | 0.585 | 0.019 | 4.236 |
| C1 | S32212 | 1.949 | 0.006 | 4.281 |
| C2 | S32213 | 0.679 | 0.006 | 4.209 |
| C3 | S32217 | 1.935 | 0.003 | 4.379 |
| C4 | S32209 | 0.609 | 0.003 | 4.298 |
| D | S32205 | 0.357 | 4.177 | 0.014 |

The antibodies belonging to Groups A and D reacted with the NTD-side domain antigen and recognized the N-terminal side of the Nu antigen. The antibodies belonging to Groups B and C reacted with the CTD-side domain antigen and recognized the C-terminal side of the Nu antigen. In both Tables 1 and 2, the combinations for which sandwiching was possible were the combination of antibodies belonging Groups B and C, and the combinations of antibodies belonging the same Group C, which suggested that using two types of antibodies that recognize the C-terminal side of the Nu antigen enabled high-sensitivity detection of the Nu antigen.

### [Example 2 Antibody-epitope analysis 2]

Antigen-immobilized ELISA was performed for more detailed epitope analysis with respect to antibodies that react with the C-terminal side of the Nu antigen.

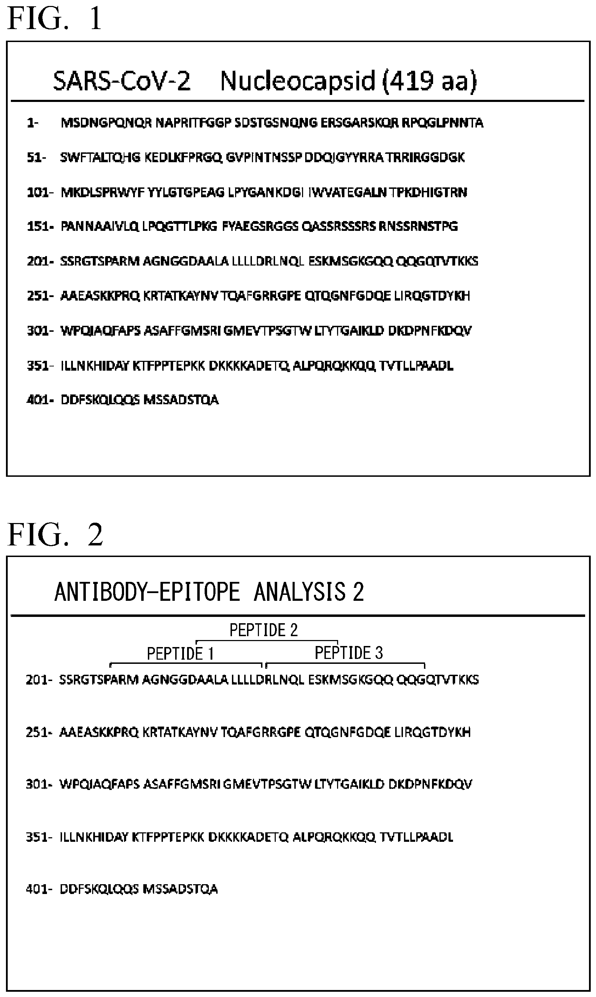

Each of synthetic peptides (10 µg/mL) having amino acid sequences corresponding to specific amino acid sequences of the Nu antigen (see FIG. 2) was dispensed into a 96-well ELISA microplate (50 µL/well) and allowed to stand at room temperature for 2 hours or 4 °C overnight. After washing 3 times with PBST (400 µL/well), a blocking solution (1% BSA-PBST) was dispensed (100 µL/well) and the resulting was allowed to stand at room temperature for 1 hour or at 4 °C overnight. After removing the blocking solution, biotin-labeled S32202, S32213, S32212, S32217, or S32209 antibody (1 µg/mL) was dispensed (50 µL/well) and the resulting was allowed to stand at room temperature for 1 hour. After washing 3 times with PBST (400 µL/well), streptavidin-HRP (×5000) was dispensed (50 µL/well) and the resulting was allowed to stand at room temperature for 1 hour. After washing 3 times with PBST (400 µL/well), an OPD coloring solution (2 mg/mL) was dispensed (50 µL/well) and the resulting was allowed to stand at room temperature for 10 minutes. The reaction stop solution was dispensed (50 µL/well), and the absorbance was measured using a plate reader (wavelength: 492 nm).

Table 4 shows the reactivity of antibodies representing the respective groups with synthetic peptides. Those that reacted are marked "+", and those that did not react are marked "-". The antibodies belonging to Groups C1 and C2 reacted with a sequence of the 388th to 405th amino acids in the nucleocapsid protein (SEQ ID NO: 48). The antibodies belonging to Groups B, C3, and C4 reacted with a sequence of the 397th to 414th amino acids (SEQ ID NO: 49).

**[Table 4]**

| | | | B | C1 | C2 | C3 | C4 |
|---|---|---|---|---|---|---|---|
| Position | Amino acid sequence | SEQ ID NO: | S32202 | S32212 | S32213 | S32217 | S32209 |
| 208-225 | ARMAGNGGDAALALLLLD | SEQ ID NO:28 | - | - | - | - | - |
| 217-234 | AALALLLLDRLNQLESKM | SEQ ID NO:29 | - | - | - | - | - |
| 226-243 | RLNQLESKMSGKGQQQQG | SEQ ID NO:30 | - | - | - | - | - |
| 235-252 | SGKGQQQQGQTVTKKSAA | SEQ ID NO:31 | - | - | - | - | - |
| 244-261 | QTVTKKSAAEASKKPRQK | SEQ ID NO:32 | - | - | - | - | - |
| 253-270 | EASKKPRQKRTATKAYNV | SEQ ID NO:33 | - | - | - | - | - |
| 262-279 | RTATKAYNVTQAFGRRGP | SEQ ID NO:34 | - | - | - | - | - |
| 271-288 | TQAFGRRGPEQTQGNFGD | SEQ ID NO:35 | - | - | - | - | - |
| 280-297 | EQTQGNFGDQELIRQGTD | SEQ ID NO:36 | - | - | - | - | - |
| 289-306 | QELIRQGTDYKHWPQIAQ | SEQ ID NO:37 | - | - | - | - | - |
| 298-315 | YKHWPQIAQFAPSASAFF | SEQ ID NO:38 | - | - | - | - | - |
| 307-324 | FAPSASAFFGMSRIGMEV | SEQ ID NO:39 | - | - | - | - | - |
| 316-333 | GMSRIGMEVTPSGTWLTY | SEQ ID NO:40 | - | - | - | - | - |
| 325-342 | TPSGTWLTYTGAIKLDDK | SEQ ID NO:41 | - | - | - | - | - |
| 334-351 | TGAIKLDDKDPNFKDQVI | SEQ ID NO:42 | - | - | - | - | - |
| 343-360 | DPNFKDQVILLNKHIDAY | SEQ ID NO:43 | - | - | - | - | - |
| 352-369 | LLNKHIDAYKTFPPTEPK | SEQ ID NO:44 | - | - | - | - | - |
| 361-378 | KTFPPTEPKKDKKKKADE | SEQ ID NO:45 | - | - | - | - | - |
| 370-387 | KDKKKKADETQALPQRQK | SEQ ID NO:46 | - | - | - | - | - |
| 379-396 | TQALPQRQKKQQTVTLLP | SEQ ID NO:47 | - | - | - | - | - |
| 388-405 | KQQTVTLLPAADLDDFSK | SEQ ID NO:48 | - | + | + | - | - |
| 397-414 | AADLDDFSKQLQQSMSSA | SEQ ID NO:49 | + | - | - | + | + |
| 406-419 | QLQQSMSSADSTQA | SEQ ID NO:50 | - | - | - | - | - |

### [Example 3 Antibody-epitope analysis 3]

Detailed epitope analyses for the S32213 and S32217 antibodies were performed using PEPperMAP (registered trademark) Peptide Microarray analysis service provided by PEPperPRINT. Tables 5 and 6 show excerpts of the analysis results of linear epitope mapping (peptide chain length of 15 amino acid residues, analyzed using 14 amino acid residue overlapping peptides), and conformational epitope mapping (peptide chain length of 7, 10, and 13 amino acid residues, analyzed using 6, 9, and 12 amino acid residue overlapping peptides, respectively). The S32213 antibody was shown to recognize LLPAA (SEQ ID NO: 26), which is a sequence of the 394th to 398th amino acids of the SARS-CoV-2 nucleocapsid protein, and the S32217 antibody was found to recognize LDDFSKQLQ (SEQ ID NO: 27), which is a sequence of the 400th to 408th amino acids of the same protein.

**[Table 5]**

| Position | Amino acid sequence | SEQ ID NO: | Reactivity with S32213 antibody |
|---|---|---|---|
| 385-391 | RQKKQQT | **SEQ ID NO**:51 | - |
| 386-392 | QKKQQTV | **SEQ ID NO**:52 | - |
| 387-393 | KKQQTVT | **SEQ ID NO**:53 | - |
| 388-394 | KQQTVTL | **SEQ ID NO**:54 | - |
| 389-395 | QQTVTLL | **SEQ ID NO**:55 | - |
| 390-396 | QTVTLLP | **SEQ ID NO**:56 | - |
| 391-397 | TVTLLPA | **SEQ ID NO**:57 | + |
| 392-398 | VT**LLPAA** | **SEQ ID NO**:58 | +++ |
| 393-399 | T**LLPAA**D | **SEQ ID NO**:59 | +++ |
| 394-400 | **LLPA**DL | **SEQ ID NO**:60 | +++ |
| 395-401 | LPAADLD | **SEQ ID NO**:61 | + |
| 396-402 | PAADLDD | **SEQ ID NO**:62 | - |
| 397-403 | AADLDDF | **SEQ ID NO**:63 | - |
| 398-404 | ADLDDFS | **SEQ ID NO**:64 | - |
| 399-405 | DLDDFSK | **SEQ ID NO**:65 | - |
| 400-406 | LDDFSKQ | **SEQ ID NO**:66 | - |

| | | | |
|---|---|---|---|
| +++ : Reacted plus high sensitivity, + : Reacted, - : Not reacted | | | |

**[Table 6]**

| Position | Amino acid sequence | **SEQ ID NO:** | Reactivity with S32217 antibody |
|---|---|---|---|
| 389-103 | QQTVTLLPAADLDDF | **SEQ ID NO**:67 | - |
| 390-404 | QTVTLLPAADLDDFS | **SEQ ID NO**:68 | - |
| 391-405 | TVTLLPAADLDDFSK | **SEQ ID NO**:69 | - |
| 392-406 | VTLLPAADLDDFSKQ | **SEQ ID NO**:70 | - |
| 393-407 | TLLPAADLDDFSKQL | **SEQ ID** NO:71 | + |
| 394-408 | LLPAAD**LDDFSKQLQ** | **SEQ ID** NO:72 | ++ |
| 395-409 | LPAAD**LDDFSKQLQ**Q | **SEQ ID** NO:73 | ++ |
| 396-410 | PAAD**LDDFSKQLQ**QS | **SEQ ID** NO:74 | ++ |
| 397-411 | AAD**LDDFSKQLQ**QSM | **SEQ ID** NO:75 | ++ |
| 398-412 | AD**LDDFSKQLQ**QSMS | **SEQ ID** NO:76 | ++ |
| 399-113 | D**LDDFSKQLQ**QSMSS | **SEQ ID** NO:77 | ++ |
| 400-414 | **LDDFSKQLQ**QSMSSA | **SEQ ID** NO:78 | +++ |
| 101-115 | DDFSKQLQQSMSSAD | **SEQ ID** NO:79 | + |
| 102-116 | DFSKQLQQSMSSADS | **SEQ ID** NO:80 | - |
| 403-417 | FSKQLQQSMSSADST | **SEQ ID** NO:81 | - |
| 404-418 | SKQLQQSMSSADSTQ | **SEQ ID** NO:82 | - |

| | | | |
|---|---|---|---|
| +++ : Reacted plus particularly high sensitivity, ++ : Reacted with high sensitivity, + : Reacted, - : Not reacted | | | |

### [Example 4 Detection of SARS-CoV-2 by immunochromatography]

An immunochromatography test strip was prepared in the same manner as in Assay Example 1. S32213 antibody was used as a labeled antibody, and S32217 antibody was used as a solid-phase antibody.

### Test Method

500 µL each of a sample diluent for rapid tester FLU/NEXT (Sekisui Medical Co. Ltd.) was dispensed into diluent tubes. Universal Transport Medium (BD) was added to SARS-CoV-2 PCR positive swab (Trina) to prepare a sample. A sample collection cotton swab stick was inserted into the sample to collect the sample, and then the sample was extracted with the diluent in the diluent tube. A sample filtration filter was attached to the diluent tube, and the entire amount was filtered. 120 µL of the above sample was added dropwise onto the test device, and 10 minutes later, the presence or absence of the test line was visually determined.

RNA was extracted from the same sample using QIAmp Viral RNA Mini Kit (QIAGEN). RT-PCR was performed following "Pathogen Detection Manual 2019-nCoV Ver. 2.9.1", National Institute of Infectious Diseases. The results are shown in Table 7. In this context, the RT-PCR results showed that 29 samples were positive and 26 samples were negative. Samples that were positive at the time of sample collection were purchased and used for the tests, some of which however became negative, presumably due to the effects of transportation, freezing and thawing, and sample dilution.

**[Table 7]**

| | | Immunochromatography in Example | | Total |
|---|---|---|---|---|
| | | Positive | Negative | |
| RT-PCR | Positive | 29 | 0 | 29 |
| | Negative | 2 | 24 | 26 |
| Total | | 31 | 24 | 55 |

The positive percent agreement with RT-PCR in the immunochromatography was 100 % (29/29 × 100). The negative percent agreement with RT-PCR in the immunochromatography was 92.3 % (24/26 × 100). The number of copies of SARS-CoV-2 per test was investigated for the samples used. All 29 positive samples contained 1600 or more copies of SARS-CoV-2 per test.

In this context, the package insert of the commercially available SARS-CoV-2 antigen detection reagents describe the positive percent agreement when measuring a sample with 1600 copies/test.

For Espline (registered trademark) SARS-CoV-2 (Fujirebio Inc.), the positive percent agreement was 92 % (12/13 × 100) with a measurement time of 30 minutes.

For QuickNavi (registered trademark)-COVID19 Ag (Denka Company Limited), the positive percent agreement was 96.3 % (26/27 × 100) with a measurement time of 15 minutes.

Thus, the immunochromatography of this example showed sensitivity comparable to or greater than those of the commercially available reagents for SARS-CoV-2 antigen detection with a shorter measurement time. Therefore, it can be said that SARS-CoV-2 can be detected rapidly and with high sensitivity by using the immunochromatography of this example.

### [Example 5 Specificity evaluation for immunochromatography]

The same test device as used in Example 4 was used. 500 µL each of the sample diluent for Rapid Tester FLU·NEXT was dispensed into diluent tubes, and the sample was extracted from two nasopharyngeal swab sticks used for the same healthy individual per diluent tube. A sample filtration filter was attached to the diluent tube, and the entire amount was filtered. The above sample was added dropwise in an amount of 120 µL per drop onto the same test device as used in Example 5, and 10 and 30 minutes later, visual and machine determinations were made. Further, RT-PCR was also performed in the same manner as in Example 5. Table 8 shows the determination results for 30 samples which were respectively derived from different donors.

**[Table 8]**

| | Determination | | |
|---|---|---|---|
| Sample | Immunochromatography in Example | | RT-PCR |
| | Measurement time 10 min | Measurement time 30 min | |
| 1 | - | - | - |
| 2 | - | - | - |
| 3 | - | - | - |
| 4 | - | - | - |
| 5 | - | - | - |
| 6 | - | - | - |
| 7 | - | - | - |
| 8 | - | - | - |
| 9 | - | - | - |
| 10 | | | |
| 11 | - | - | - |
| 12 | - | - | - |
| 13 | - | - | - |
| 14 | - | - | - |
| 15 | - | - | - |
| 16 | - | - | - |
| 17 | - | - | - |
| 18 | - | - | - |
| 19 | - | - | - |
| 20 | - | - | - |
| 21 | - | - | - |
| 22 | - | - | - |
| 23 | - | - | - |
| 24 | - | - | - |
| 25 | - | - | - |
| 26 | - | - | - |
| 27 | - | - | - |
| 28 | - | - | - |
| 29 | - | - | - |
| 30 | - | - | - |

All samples 1 to 30 were found to be negative by RT-PCR. In the determination performed 10 minutes after starting the immunochromatography in the example, all the samples were again found to be negative. Further, all the samples were also found to be negative in the determination performed 30 minutes after starting the immunochromatography in the example, which exceeded the predetermined measurement time for the immunochromatography in the example. Thus, the immunochromatography in this example showed good specificity.

### [Example 6 Analysis of amino acid sequence of monoclonal antibody]

The amino acid sequences of the heavy chain variable region and light chain variable region of the S32213 antibody, S32217 antibody, and S32223 antibody were analyzed using the antibody variable region analysis of Kazusa DNA Research Institute, a public interest incorporated foundation. As a result, the amino acid sequences of the heavy chain variable region and the light chain variable region were respectively found to be as shown in Table 9 below.

**[Table 9]**

| Name of antibody | Heavy chain or light chain | CDR | Amino acid sequence | SEQ ID NO: |
|---|---|---|---|---|
| S32213 | Heavy chain | **CDR1** | GFSLSTSGMG | SEQ ID NO:4 |
| | | **CDR2** | IYWDDDK | SEQ ID NO:5 |
| | | **CDR3** | ARRDYGYNFDY | SEQ ID NO:6 |
| | Light chain | **CDR1** | SSVSSTY | SEQ ID NO:7 |
| | | **CDR2** | STS | SEQ ID NO:8 |
| | | **CDR3** | HQWSSYPPT | SEQ ID NO:9 |
| S32217 | Heavy chain | **CDR1** | GFTFSDYY | SEQ ID NO:10 |
| | | **CDR2** | TTDGDNYT | SEQ ID NO:11 |
| | | **CDR3** | ARDGNYYASSPFTY | SEQ ID NO:12 |
| | Light chain | **CDR1** | TGAVTTSNY | SEQ ID NO:13 |
| | | **CDR2** | GTN | SEQ ID NO: 14 |
| | | **CDR3** | ALWYSNHWV | SEQ ID NO:15 |
| S32223 | Heavy chain | **CDR1** | GFTFSDYY | SEQ ID NO: 16 |
| | | **CDR2** | ISDGYSYT | SEQ ID NO:17 |
| | | **CDR3** | ARDQDYFGSSLAY | SEQ ID NO:18 |
| | Light chain | **CDR1** | TGA VTTSNY | SEQ ID NO:19 |
| | | **CDR2** | GTN | SEQ ID NO:20 |
| | | **CDR3** | ALWYSNRWV | SEQ ID NO:21 |

As in the case of the S32217 antibody, the S32223 antibody also belongs to the Group C3 in the antibody classification by sandwich ELISA in Assay Example 1. The amino acid sequences of CDR1 to CDR3 in the heavy chain variable region and light chain variable region of the S32223 antibody respectively have high identity with the amino acid sequences of CDR1 to CDR3 in the heavy chain variable region and light chain variable region of the S32217 antibody. Therefore, the S32223 antibody is presumed to have similar reactivity to the SARS-CoV-2 antigen as the S32217 antibody.

### [Example 7 Comparison with commercially available SARS-CoV-2 antigen detection reagents]

A SARS-CoV-2 positive sample was appropriately diluted with a sample transport medium, and the resulting was added to each of a sample diluent for rapid tester FLU/NEXT (Sekisui Medical Co., Ltd.), Espline (registered trademark) SARS-CoV-2 sample treatment solution (Fujirebio), and QuickNavi (registered trademark) sample suspension (for COVID19 Ag) (Denka Company Limited) to prepare samples. The above samples were measured with the immunochromatography test strip used in Example 4, Espline (registered trademark) SARS-CoV-2 (Fujirebio) and QuickNavi (registered trademark)-COVID19 Ag (Denka Company Limited), each of which is an existing SARS-CoV-2 measurement reagents.

The measurement results are shown in Table 10.

**[Table 10]**

| Sample | Dilution ratio | Example 4 | Espline | QuickNavi |
|---|---|---|---|---|
| | | 10min | 30min | lSmin |
| Sample 1 | 800-fold | + | + | - |
| | 1600-fold | + | - | - |
| | 3200-fold | - | NT | NT |
| Sample 2 | 100-fold | + | + | +/- |
| | 200-fold | NT | NT | - |
| | 800-fold | NT | + | NT |
| | 1600-fold | + | - | - |
| | 3200-fold | + | - | NT |
| | 6400-fold | + | NT | NT |
| | 12800-fold | - | NT | NT |
| Sample 3 | 50-fold | + | + | + |
| | 100-fold | NT | NT | - |
| | 400-fold | NT | + | - |
| | 800-fold | + | - | - |
| | 1600-fold | + | - | - |
| | 3200-fold | - | NT | NT |
| Sample 4 | 50-fold | + | + | +/- |
| | 100-fold | NT | NT | - |
| | 200-fold | + | + | NT |
| | 400-fold | - | - | NT |

The above results demonstrate that a highly sensitive and rapid SARS-CoV-2 measurement system has been established by using two monoclonal antibodies that bind to a peptide fragment having the amino acid sequence represented by SEQ ID NO: 1.

That is, it has been shown that the measurement reagent of the present invention exhibits comparable or superior sensitivity to the commercially available SARS-CoV-2 antigen detection reagents in a shorter measurement time, and enables a rapid and high-sensitivity detection of SARS-CoV-2.

### [List of Sequences]

**[Table 11]**

| SEQ ID NO: | Amino acid sequence (N terminus → C terminus) |
|---|---|
| 1 | KQQTVTLLPAADLDDFSKQLQQSMSSA |
| 2 | KQQTVTLLPAADLDDFSK |
| 3 | AADLDDFSKQLQQSMSSA |
| 4 | GFSLSTSGMG |
| 5 | IYWDDDK |
| 6 | ARRDYGYNFDY |
| 7 | SSVSSTY |
| 8 | STS |
| 9 | HQWSSYPPT |
| 10 | GFTFSDYY |
| 11 | ITDGDNYT |
| 12 | ARDGNYYASSPFTY |
| 13 | TGAVTTSNY |
| 14 | GTN |
| 15 | ALWYSNHWV |
| 16 | GFTFSDYY |
| 17 | ISDGYSYT |
| 18 | ARDQDYFGSSLAY |
| 19 | TGAVTTSNY |
| 20 | GTN |
| 21 | ALWYSNRWV |

**[Table 12]**

| SEQ ID NO: | Amino acid sequence (N terminus → C terminus) |
|---|---|
| 22 | MSDNGPQNQR NAPRITFGGP SDSTGSNQNG ERSGARSKQR RPQGLPNNTA SWFTALTQHG KEDLKFPRGQ GVPINTNSSP DDQIGYYRRA TRRIRGGDGK MKDLSPRWYF YYLGTGPEAG LPYGANKDGI IWVATEGALN TPKDHIGTRN PANNAAIVLQ LPQGTTLPKG FYAEGSRGGS QASSRSSSRS RNSSRNSTPG SSRGTSPARM AGNGGDAALA LLLLDRLNQL ESKMSGKGQQ QQGQTVTKKS AAEASKKPRQ KRTATKAYNV TQAFGRRGPE QTQGNFGDQE LIRQGTDYKH WPQIAQFAPS ASAFFGMSRI GMEVTPSGTW LTYTGAIKLD DKDPNFKDQV ILLNKHIDAY KTFPPTEPKK DKKKKADETQ ALPQRQKKQQ TVTLLPAADL DDFSKQLQQS MSSADSTQA |
| 23 | MSDNGPQNQR NAPRITFGGP SDSTGSNQNG ERSGARSKQR RPQGLPNNTA SWFTALTQHG KEDLKFPRGQ GVPINTNSSP DDQIGYYRRA TRRIRGGDGK MKDLSPRWYF YYLGTGPEAG LPYGANKDGI IWVATEGALN TPKDHIGTRN PANNAAIVLQ LPQGTTLPKG FYAE |
| 24 | GSRGGSQASS RSSSRSRNSS RNSTPGSSRG TSPARMAGNG GDAALALLLL DRLNQLESKM SGKGQQQQGQ TVT |
| 25 | KKSAAEASKK PRQKRTATKA YNVTQAFGRR GPEQTQGNFG DQELIRQGTD YKHWPQIAQF APSASAFFGM SRIGMEVTPS GTWLTYTGAI KLDDKDPNFK DQVILLNKHI DAYKTFPPTE PKKDKKKKAD ETQALPQRQK KQQTVTLLPA ADLDDFSKQL QQSMSSADST QA |

**[Table 13]**

| SEQ ID NO: | Amino acid sequence (N terminus → C terminus) |
|---|---|
| 26 | LLPAA |
| 27 | LDDFSKQLQ |
| 28 | ARMAGNGGDAALALLLLD |
| 29 | TAALALLLLDRLNQLESKM |
| 30 | RLNQLESKMSGKGQQQQG |
| 31 | SGKGQQQQGQTVTKKSAA |
| 32 | QTVTKKSAAEASKKPRQK |
| 33 | EASKKPRQKRTATKAYNV |
| 34 | RTATKAYNVTQAFGRRGP |
| 35 | TQAFGRRGPEQTQGNFGD |
| 36 | EQTQGNFGDQELIRQGTD |
| 37 | QELIRQGTDYKHWPQIAQ |
| 38 | YKHWPQIAQFAPSASAFF |
| 39 | FAPSASAFFGMSRIGMEV |
| 40 | GMSRIGMEVTPSGTWLTY |
| 41 | TPSGTWLTYTGAIKLDDK |
| 42 | TGAIKLDDKDPNFKDQVI |
| 43 | DPNFKDQVILLNKHIDAY |
| 44 | LLNKHIDAYKITFPPTEPK |
| 45 | KTFPPTEPKKDKKKKADE |
| 46 | KDKKKKADETQALPQRQK |
| 47 | TQALPQRQKKQQTVTLLP |
| 48 | KQQTVTLLPAADLDDFSK |
| 49 | AADLDDFSKQLQQSMSSA |
| 50 | QLQQSMSSADSTQA |

**[Table 14]**

| SEQ ID NO: | Amino acid sequence (N terminus → C terminus) |
|---|---|
| 51 | RQKKQQT |
| 52 | QKKQQTV |
| 53 | KKQQTVT |
| 54 | KQQTVTL |
| 55 | QQTVTLL |
| 56 | QTVTLLP |
| 57 | TVTLLPA |
| 58 | VTLLPAA |
| 59 | TLLPAAD |
| 60 | LLPAADL |
| 61 | LPAADLD |
| 62 | PAADLDD |
| 63 | AADIDDF |
| 64 | ADLDDFS |
| 65 | DLDDESK |
| 66 | LDDFSKQ |

**[Table 15]**

| SEQ ID NO: | Amino acid sequence (N terminus → C terminus) |
|---|---|
| 67 | QQTVTLLPAADLDDF |
| 68 | QTVTLLPAADLDDFS |
| 69 | TVTLLPAADLDDFSK |
| 70 | VTLLPAADLDDESKQ |
| 71 | TLLPAADLDDFSKQL |
| 72 | LLPAADLDDESKQLQ |
| 73 | LPAADLDDFSKQLQQ |
| 74 | PAADLDDFSKQLQQS |
| 75 | AADLDDFSKQLQQSM |
| 76 | ADLDDFSKQLQQSMS |
| 77 | DLDDFSKQLQQSMSS |
| 78 | LDDFSKQLQQSMSSA |
| 79 | DDFSKQLQQSMSSAD |
| 80 | DFSKQLQQSMSSADS |
| 81 | FSKQLQQSMSSADST |
| 82 | SKQLQQSMSSADSTQ |

### [Industrial Applicability]

The present invention can provide a SARS-CoV-2 immunoassay kit and a SARS-CoV-2 immunoassay method that enable highly sensitive and rapid immunoassay, as well as a monoclonal antibody or an antibody fragment thereof that can be used therein.

## Claims

1. An in-vitro immunoassay method for assaying SARS-CoV-2 in a biological sample, comprising using two types of monoclonal antibodies or antibody fragments thereof that bind to a peptide fragment having 30 or less consecutive amino acids in a nucleocapsid protein of SARS-CoV-2, wherein the two types of monoclonal antibodies or antibody fragments thereof recognize different epitopes, and
wherein the peptide fragment having 30 or less consecutive amino acids is a peptide fragment having an amino acid sequence represented by KQQTVTLLPAADLDDFSKQLQQSMSSA (SEQ ID NO: 1).

2. The immunoassay method according to claim 1, wherein the biological sample is a respiratory secretion.

3. The immunoassay method according to claim 1 or 2, wherein the two types of monoclonal antibodies or antibody fragments thereof are a first monoclonal antibody or an antibody fragment thereof and a second monoclonal antibody or an antibody fragment thereof, wherein the first monoclonal antibody or the antibody fragment thereof has a labeling substance indirectly or directly bound thereto, and the second monoclonal antibody or the antibody fragment thereof is indirectly or directly bound to a solid phase.

4. The immunoassay method according to claim 3, comprising:
(1) a step of contacting a biological sample with the first monoclonal antibody or the antibody fragment thereof having the labeling substance bound thereto to form a first complex;
(2) a step of contacting the first complex with the second monoclonal antibody or the antibody fragment thereof to form a second complex; and
(3) a step of measuring a signal attributable to the labeling substance.

5. The immunoassay method according to claim 3 or 4, wherein the first monoclonal antibody or the antibody fragment thereof is a monoclonal antibody or an antibody fragment thereof that recognizes an epitope in the amino acid sequence represented by KQQTVTLLPAADLDFSK (SEQ ID NO: 2), and the second monoclonal antibody or the antibody fragment thereof is a monoclonal antibody or an antibody fragment thereof that recognizes an epitope in the amino acid sequence represented by AADLDDFSKQLQQSMSSA (SEQ ID NO: 3).

6. The immunoassay method according to any one of claims 1 to 4, wherein one of the monoclonal antibodies recognizes an epitope in the amino acid sequence represented by KQQTVTLLPAADLDDFSK (SEQ ID NO: 2), while the other monoclonal antibody recognizes an epitope in the amino acid sequence represented by AADLDDFSKQLQQSMSSA (SEQ ID NO: 3).

7. The immunoassay method according to any one of claims 1 to 5, wherein the two types of monoclonal antibodies or antibody fragments thereof are, respectively, a monoclonal antibody or an antibody fragment thereof that recognizes an amino acid sequence represented by LLPAA (SEQ ID NO: 26) as an epitope, and a monoclonal antibody or an antibody fragment thereof that recognizes an epitope in an amino acid sequence represented by LDDFSKQLQ (SEQ ID NO: 27).

8. The immunoassay method according to any one of claims 1 to 7, which is immunochromatography or ELISA.

9. The immunoassay method according to any one of claims 1 to 8, wherein the two types of monoclonal antibodies or the antibody fragments thereof are selected from the group consisting of:
(1) an antibody or an antibody fragment thereof that comprises a heavy chain variable region comprising CDR1 having an amino acid sequence of SEQ ID NO: 4, CDR2 having an amino acid sequence of SEQ ID NO: 5, and CDR3 having an amino acid sequence of SEQ ID NO: 6, and a light chain variable region comprising CDR1 having an amino acid sequence of SEQ ID NO: 7, CDR2 having an amino acid sequence of SEQ ID NO: 8, and CDR3 having an amino acid sequence of SEQ ID NO: 9;
(2) an antibody or an antibody fragment thereof that comprises a heavy chain variable region comprising CDR1 having an amino acid sequence of SEQ ID NO: 10, CDR2 having an amino acid sequence of SEQ ID NO: 11, and CDR3 having an amino acid sequence of SEQ ID NO: 12, and a light chain variable region comprising CDR1 having an amino acid sequence of SEQ ID NO: 13, CDR2 having an amino acid sequence of SEQ ID NO: 14, and CDR3 having an amino acid sequence of SEQ ID NO: 15; and
(3) an antibody or an antibody fragment thereof that comprises a heavy chain variable region comprising CDR1 having an amino acid sequence of SEQ ID NO: 16, CDR2 having an amino acid sequence of SEQ ID NO: 17, and CDR3 having an amino acid sequence of SEQ ID NO: 18, and a light chain variable region comprising CDR1 having an amino acid sequence of SEQ ID NO: 19, CDR2 having an amino acid sequence of SEQ ID NO: 20, and CDR3 having an amino acid sequence of SEQ ID NO: 21.

10. An immunoassay kit for detecting SARS-CoV-2 in a biological sample, comprising two types of monoclonal antibodies or antibody fragments thereof that bind to a peptide fragment having 30 or less consecutive amino acids in a nucleocapsid protein of SARS-CoV-2, wherein the two types of monoclonal antibodies or antibody fragments thereof recognize different epitopes, and
wherein the peptide fragment having 30 or less consecutive amino acids is a peptide fragment having an amino acid sequence represented by KQQTVTLLPAADLDDFSKQLQQSMSSA (SEQ ID NO: 1).

11. The immunoassay kit according to claim 10, wherein the biological sample is a respiratory secretion.

12. The immunoassay kit according to claim 10 or 11, wherein the two types of monoclonal antibodies or antibody fragments thereof are a first monoclonal antibody or an antibody fragment thereof and a second monoclonal antibody or an antibody fragment thereof, wherein the first monoclonal antibody or the antibody fragment thereof has a labeling substance indirectly or directly bound thereto, and the second monoclonal antibody or the antibody fragment thereof is indirectly or directly bound to a solid phase.

13. The immunoassay kit according to claim 12, wherein the first monoclonal antibody or the antibody fragment thereof is a monoclonal antibody or an antibody fragment thereof that recognizes an epitope in the amino acid sequence represented by KQQTVTLLPAADLDFSK (SEQ ID NO: 2), and the second monoclonal antibody or the antibody fragment thereof is a monoclonal antibody or an antibody fragment thereof that recognizes an epitope in the amino acid sequence represented by AADLDDFSKQLQQSMSSA (SEQ ID NO: 3).

14. The immunoassay kit according to any one of claims 10 to 12, wherein one of the monoclonal antibodies recognizes an epitope in the amino acid sequence represented by KQQTVTLLPAADLDDFSK (SEQ ID NO: 2), while the other monoclonal antibody recognizes an epitope in the amino acid sequence represented by AADLDDFSKQLQQSMSSA (SEQ ID NO: 3).

15. The immunoassay kit according to any one of claims 10 to 13, wherein the two types of monoclonal antibodies or antibody fragments thereof are, respectively, a monoclonal antibody or an antibody fragment thereof that recognizes an amino acid sequence represented by LLPAA (SEQ ID NO: 26) as an epitope, and a monoclonal antibody or an antibody fragment thereof that recognizes an epitope in an amino acid sequence represented by LDDFSKQLQ (SEQ ID NO: 27).

16. The immunoassay kit according to any one of claims 10 to 15, which is a kit for immunochromatography or ELISA.

17. The immunoassay kit according to any one of claims 10 to 16, wherein the two types of monoclonal antibodies or the antibody fragments thereof are selected from the group consisting of:
(1) an antibody or an antibody fragment thereof that comprises a heavy chain variable region comprising CDR1 having an amino acid sequence of SEQ ID NO: 4, CDR2 having an amino acid sequence of SEQ ID NO: 5, and CDR3 having an amino acid sequence of SEQ ID NO: 6, and a light chain variable region comprising CDR1 having an amino acid sequence of SEQ ID NO: 7, CDR2 having an amino acid sequence of SEQ ID NO: 8, and CDR3 having an amino acid sequence of SEQ ID NO: 9;
(2) an antibody or an antibody fragment thereof that comprises a heavy chain variable region comprising CDR1 having an amino acid sequence of SEQ ID NO: 10, CDR2 having an amino acid sequence of SEQ ID NO: 11, and CDR3 having an amino acid sequence of SEQ ID NO: 12, and a light chain variable region comprising CDR1 having an amino acid sequence of SEQ ID NO: 13, CDR2 having an amino acid sequence of SEQ ID NO: 14, and CDR3 having an amino acid sequence of SEQ ID NO: 15; and
(3) an antibody or an antibody fragment thereof that comprises a heavy chain variable region comprising CDR1 having an amino acid sequence of SEQ ID NO: 16, CDR2 having an amino acid sequence of SEQ ID NO: 17, and CDR3 having an amino acid sequence of SEQ ID NO: 18, and a light chain variable region comprising CDR1 having an amino acid sequence of SEQ ID NO: 19, CDR2 having an amino acid sequence of SEQ ID NO: 20, and CDR3 having an amino acid sequence of SEQ ID NO: 21.

## Patentansprüche

1. In vitro-Immunassay-Verfahren zum Nachweis von SARS-CoV-2 in einer biologischen Probe, umfassend die Verwendung zweier Arten von monoklonalen Antikörpern oder Antikörperfragmenten davon, die an ein Peptidfragment mit 30 oder weniger aufeinanderfolgenden Aminosäuren in einem Nukleokapsidprotein von SARS-CoV-2 binden, wobei die beiden Arten von monoklonalen Antikörpern oder Antikörperfragmenten davon unterschiedliche Epitope erkennen, und
wobei das Peptidfragment mit 30 oder weniger aufeinanderfolgenden Aminosäuren ein Peptidfragment mit der Aminosäuresequenz KQQTVTLLPAADLDDFSKQLQQSMSSA (SEQ ID NO: 1) ist.

2. Immunassay-Verfahren nach Anspruch 1, wobei es sich bei der biologischen Probe um ein Atemwegssekret handelt.

3. Immunassay-Verfahren nach Anspruch 1 oder 2, wobei es sich bei den beiden Arten von monoklonalen Antikörpern oder Antikörperfragmenten davon um einen ersten monoklonalen Antikörper oder ein Antikörperfragment davon und einen zweiten monoklonalen Antikörper oder ein Antikörperfragment davon handelt, wobei an den ersten monoklonalen Antikörper oder das Antikörperfragment davon eine Markierungssubstanz indirekt oder direkt gebunden ist und der zweite monoklonale Antikörper oder das Antikörperfragment davon indirekt oder direkt an eine feste Phase gebunden ist.

4. Immunassay-Verfahren nach Anspruch 3, umfassend:
(1) einen Schritt des Inkontaktbringens einer biologischen Probe mit dem ersten monoklonalen Antikörper oder dem Antikörperfragment davon, an den die Markierungssubstanz gebunden ist, zur Bildung eines ersten Komplexes;
(2) einen Schritt des Inkontaktbringens des ersten Komplexes mit dem zweiten monoklonalen Antikörper oder dem Antikörperfragment davon zur Bildung eines zweiten Komplexes; und
(3) einen Schritt des Messens eines Signals, das auf die Markierungssubstanz zurückzuführen ist.

5. Immunassay-Verfahren nach Anspruch 3 oder 4, wobei der erste monoklonale Antikörper oder das Antikörperfragment davon ein monoklonaler Antikörper oder ein Antikörperfragment davon ist, der ein Epitop in der durch KQQTVTLLPAADLDFSK (SEQ ID NO: 2) dargestellten Aminosäuresequenz erkennt, und der zweite monoklonale Antikörper oder das Antikörperfragment davon ein monoklonaler Antikörper oder ein Antikörperfragment davon ist, der ein Epitop in der durch AADLDDFSKQLQQSMSSA (SEQ ID NO: 3) dargestellten Aminosäuresequenz erkennt.

6. Immunassay-Verfahren nach einem der Ansprüche 1 bis 4, wobei einer der monoklonalen Antikörper ein Epitop in der durch KQQTVTLLPAADLDDFSK (SEQ ID NO: 2) dargestellten Aminosäuresequenz erkennt, während der andere monoklonale Antikörper ein Epitop in der durch AADLDDFSKQLQQSMSSA (SEQ ID NO: 3) dargestellten Aminosäuresequenz erkennt.

7. Immunassay-Verfahren nach einem der Ansprüche 1 bis 5, wobei es sich bei den beiden Arten von monoklonalen Antikörpern oder Antikörperfragmenten davon jeweils um einen monoklonalen Antikörper oder ein Antikörperfragment davon handelt, der/das die durch LLPAA (SEQ ID NO: 26) dargestellte Aminosäuresequenz als Epitop erkennt, und um einen monoklonalen Antikörper oder ein Antikörperfragment davon, der/das ein Epitop in der durch LDDFSKQLQ (SEQ ID NO: 27) dargestellten Aminosäuresequenz erkennt.

8. Immunassay-Verfahren nach einem der Ansprüche 1 bis 7, bei dem es sich um Immunochromatographie oder ELISA handelt.

9. Immunassay-Verfahren nach einem der Ansprüche 1 bis 8, wobei die beiden Arten von monoklonalen Antikörpern oder Antikörperfragmenten davon ausgewählt sind aus der Gruppe bestehend aus:
(1) einem Antikörper oder einem Antikörperfragment davon, der eine variable Region der schweren Kette umfasst, umfassend CDR1 mit der Aminosäuresequenz der SEQ ID NO: 4, CDR2 mit der Aminosäuresequenz der SEQ ID NO: 5 und CDR3 mit der Aminosäuresequenz der SEQ ID NO: 6, und eine variable Region der leichten Kette, umfassend CDR1 mit der Aminosäuresequenz der SEQ ID NO: 7, CDR2 mit der Aminosäuresequenz der SEQ ID NO: 8 und CDR3 mit der Aminosäuresequenz der SEQ ID NO: 9;
(2) einem Antikörper oder einem Antikörperfragment davon, der eine variable Region der schweren Kette umfasst, umfassend CDR1 mit der Aminosäuresequenz der SEQ ID NO: 10, CDR2 mit der Aminosäuresequenz der SEQ ID NO: 11 und CDR3 mit der Aminosäuresequenz der SEQ ID NO: 12, und eine variable Region der leichten Kette, umfassend CDR1 mit der Aminosäuresequenz der SEQ ID NO: 13, CDR2 mit der Aminosäuresequenz der SEQ ID NO: 14 und CDR3 mit der Aminosäuresequenz der SEQ ID NO: 15; und
(3) einem Antikörper oder einem Antikörperfragment davon, der eine variable Region der schweren Kette umfasst, umfassend CDR1 mit der Aminosäuresequenz der SEQ ID NO: 16, CDR2 mit der Aminosäuresequenz der SEQ ID NO: 17 und CDR3 mit der Aminosäuresequenz der SEQ ID NO: 18, und eine variable Region der leichten Kette, umfassend CDR1 mit der Aminosäuresequenz der SEQ ID NO: 19, CDR2 mit der Aminosäuresequenz der SEQ ID NO: 20 und CDR3 mit der Aminosäuresequenz der SEQ ID NO: 21.

10. Immunoassay-Kit zum Nachweis von SARS-CoV-2 in einer biologischen Probe, umfassend zwei Arten von monoklonalen Antikörpern oder Antikörperfragmenten davon, die an ein Peptidfragment mit 30 oder weniger aufeinanderfolgenden Aminosäuren in einem Nukleokapsidprotein von SARS-CoV-2 binden, wobei die beiden Arten von monoklonalen Antikörpern oder Antikörperfragmenten davon unterschiedliche Epitope erkennen und
wobei das Peptidfragment mit 30 oder weniger aufeinanderfolgenden Aminosäuren ein Peptidfragment mit der Aminosäuresequenz KQQTVTLLPAADLDDFSKQLQQSMSSA (SEQ ID NO: 1) ist.

11. Immunoassay-Kit nach Anspruch 10, wobei die biologische Probe ein Atemwegssekret ist.

12. Immunoassay-Kit nach Anspruch 10 oder 11, wobei die beiden Arten von monoklonalen Antikörpern oder Antikörperfragmenten davon ein erster monoklonaler Antikörper oder ein Antikörperfragment davon und ein zweiter monoklonaler Antikörper oder ein Antikörperfragment davon sind, wobei an den ersten monoklonalen Antikörper oder das Antikörperfragment davon eine Markierungssubstanz indirekt oder direkt gebunden ist und der zweite monoklonale Antikörper oder das Antikörperfragment davon indirekt oder direkt an eine feste Phase gebunden ist.

13. Immunassay-Kit nach Anspruch 12, wobei der erste monoklonale Antikörper oder das Antikörperfragment davon ein monoklonaler Antikörper oder ein Antikörperfragment davon ist, der ein Epitop in der durch KQQTVTLLPAADLDFSK (SEQ ID NO: 2) dargestellten Aminosäuresequenz erkennt, und der zweite monoklonale Antikörper oder das Antikörperfragment davon ein monoklonaler Antikörper oder ein Antikörperfragment davon ist, der ein Epitop in der durch AADLDDFSKQLQQSMSSA (SEQ ID NO: 3) dargestellten Aminosäuresequenz erkennt.

14. Immunoassay-Kit nach einem der Ansprüche 10 bis 12, wobei einer der monoklonalen Antikörper ein Epitop in der durch KQQTVTLLPAADLDDFSK (SEQ ID NO: 2) dargestellten Aminosäuresequenz erkennt, während der andere monoklonale Antikörper ein Epitop in der durch AADLDDFSKQLQQSMSSA (SEQ ID NO: 3) dargestellten Aminosäuresequenz erkennt.

15. Immunoassay-Kit nach einem der Ansprüche 10 bis 13, wobei es sich bei den beiden Arten von monoklonalen Antikörpern oder Antikörperfragmenten davon jeweils um einen monoklonalen Antikörper oder ein Antikörperfragment davon handelt, der/das die durch LLPAA (SEQ ID NO: 26) dargestellte Aminosäuresequenz als Epitop erkennt, und um einen monoklonalen Antikörper oder ein Antikörperfragment davon, der/das ein Epitop in der durch LDDFSKQLQ (SEQ ID NO: 27) dargestellten Aminosäuresequenz erkennt.

16. Immunoassay-Kit nach einem der Ansprüche 10 bis 15, bei dem es sich um ein Kit für Immunochromatographie oder ELISA handelt.

17. Immunoassay-Kit nach einem der Ansprüche 10 bis 16, wobei die beiden Arten von monoklonalen Antikörpern oder deren Antikörperfragmenten ausgewählt sind aus der Gruppe bestehend aus:
(1) einem Antikörper oder einem Antikörperfragment davon, der eine variable Region der schweren Kette umfasst, umfassend CDR1 mit der Aminosäuresequenz der SEQ ID NO: 4, CDR2 mit der Aminosäuresequenz der SEQ ID NO: 5 und CDR3 mit der Aminosäuresequenz der SEQ ID NO: 6, und eine variable Region der leichten Kette, umfassend CDR1 mit der Aminosäuresequenz der SEQ ID NO: 7, CDR2 mit der Aminosäuresequenz der SEQ ID NO: 8 und CDR3 mit der Aminosäuresequenz der SEQ ID NO: 9;
(2) einem Antikörper oder einem Antikörperfragment davon, der eine variable Region der schweren Kette umfasst, umfassend CDR1 mit der Aminosäuresequenz der SEQ ID NO: 10, CDR2 mit der Aminosäuresequenz der SEQ ID NO: 11 und CDR3 mit der Aminosäuresequenz der SEQ ID NO: 12, und eine variable Region der leichten Kette, umfassend CDR1 mit der Aminosäuresequenz der SEQ ID NO: 13, CDR2 mit der Aminosäuresequenz der SEQ ID NO: 14 und CDR3 mit der Aminosäuresequenz der SEQ ID NO: 15; und
(3) einem Antikörper oder einem Antikörperfragment davon, der eine variable Region der schweren Kette umfasst, umfassend CDR1 mit der Aminosäuresequenz der SEQ ID NO: 16, CDR2 mit der Aminosäuresequenz der SEQ ID NO: 17 und CDR3 mit der Aminosäuresequenz der SEQ ID NO: 18, und eine variable Region der leichten Kette, umfassend CDR1 mit der Aminosäuresequenz der SEQ ID NO: 19, CDR2 mit der Aminosäuresequenz der SEQ ID NO: 20 und CDR3 mit der Aminosäuresequenz der SEQ ID NO: 21.

## Revendications

1. Procédé de dosage immunologique in vitro pour doser le SARS-CoV-2 dans un échantillon biologique, comportant d'utiliser deux types d'anticorps monoclonaux ou de fragments d'anticorps de ceux-ci qui se lient à un fragment de peptide ayant 30 acides aminés consécutifs ou moins dans une protéine nucléocapsidique du SARS-CoV-2, dans lequel les deux types d'anticorps monoclonaux ou de fragments d'anticorps de ceux-ci reconnaissent des épitopes différents, et
dans lequel le fragment de peptide ayant 30 acides aminés consécutifs ou moins est un fragment de peptide ayant une séquence d'acides aminés représentée par KQQTVTLLPAADLDDFSKQLQQSMSSA (SÉQ ID N° : 1).

2. Procédé de dosage immunologique selon la revendication 1, dans lequel l'échantillon biologique est une sécrétion respiratoire.

3. Procédé de dosage immunologique selon la revendication 1 ou 2, dans lequel les deux types d'anticorps monoclonaux ou de fragments d'anticorps de ceux-ci sont un premier anticorps monoclonal ou un fragment d'anticorps de celui-ci et un second anticorps monoclonal ou un fragment d'anticorps de celui-ci, dans lequel le premier anticorps monoclonal ou le fragment d'anticorps de celui-ci a une substance de marquage indirectement ou directement liée à celui-ci, et le second anticorps monoclonal ou le fragment d'anticorps de celui-ci est indirectement ou directement lié à une phase solide.

4. Procédé de dosage immunologique selon la revendication 3, comportant :
(1) une étape consistant à mettre en contact un échantillon biologique avec le premier anticorps monoclonal ou le fragment d'anticorps de celui-ci auquel est liée la substance de marquage pour former un premier complexe ;
(2) une étape consistant à mettre en contact le premier complexe avec le second anticorps monoclonal ou le fragment d'anticorps de celui-ci pour former un second complexe ; et
(3) une étape consistant à mesurer un signal imputable à la substance de marquage.

5. Procédé de dosage immunologique selon la revendication 3 ou 4, dans lequel le premier anticorps monoclonal ou le fragment d'anticorps de celui-ci est un anticorps monoclonal ou un fragment d'anticorps de celui-ci qui reconnaît un épitope dans la séquence d'acides aminés représentée par KQQTVTLLPAADLDFSK (SÉQ ID N° : 2), et le second anticorps monoclonal ou le fragment d'anticorps de celui-ci est un anticorps monoclonal ou un fragment d'anticorps de celui-ci qui reconnaît un épitope dans la séquence d'acides aminés représentée par AADLDDFSKQLQQSMSSA (SÉQ ID N° : 3).

6. Procédé de dosage immunologique selon l'une quelconque des revendications 1 à 4, dans lequel l'un des anticorps monoclonaux reconnaît un épitope dans la séquence d'acides aminés représentée par KQQTVTLLPAADLDDFSK (SÉQ ID N° : 2), alors que l'autre anticorps monoclonal reconnaît un épitope dans la séquence d'acides aminés représentée par AADLDDFSKQLQQSMSSA (SÉQ ID N° : 3).

7. Procédé de dosage immunologique selon l'une quelconque des revendications 1 à 5, dans lequel les deux types d'anticorps monoclonaux ou de fragments d'anticorps de ceux-ci sont, respectivement, un anticorps monoclonal ou un fragment d'anticorps de celui-ci qui reconnaît une séquence d'acides aminés représentée par LLPAA (SÉQ ID N° : 26) comme un épitope, et un anticorps monoclonal ou un fragment d'anticorps de celui-ci qui reconnaît un épitope dans une séquence d'acides aminés représentée par LDDFSKQLQ (SÉQ ID N° : 27).

8. Procédé de dosage immunologique selon l'une quelconque des revendications 1 à 7, qui est une immunochromatographie ou une ELISA.

9. Procédé de dosage immunologique selon l'une quelconque des revendications 1 à 8, dans lequel les deux types d'anticorps monoclonaux ou de fragments d'anticorps de ceux-ci sont choisis parmi le groupe constitué de :
(1) un anticorps ou un fragment d'anticorps de celui-ci qui comporte une région variable d'une chaîne lourde comportant CDR1 ayant une séquence d'acides aminés de la SÉQ ID N° : 4, CDR2 ayant une séquence d'acides aminés de la SÉQ ID N° : 5, et CDR3 ayant une séquence d'acides aminés de la SÉQ ID N° : 6, et une région variable d'une chaîne légère comportant CDR1 ayant une séquence d'acides aminés de la SÉQ ID N° : 7, CDR2 ayant une séquence d'acides aminés de la SÉQ ID N° : 8, et CDR3 ayant une séquence d'acides aminés de la SÉQ ID N° : 9 ;
(2) un anticorps ou un fragment d'anticorps de celui-ci qui comporte une région variable d'une chaîne lourde comportant CDR1 ayant une séquence d'acides aminés de la SÉQ ID N° : 10, CDR2 ayant une séquence d'acides aminés de la SÉQ ID N° : 11, et CDR3 ayant une séquence d'acides aminés de la SÉQ ID N° : 12, et une région variable d'une chaîne légère comportant CDR1 ayant une séquence d'acides aminés de la SÉQ ID N° : 13, CDR2 ayant une séquence d'acides aminés de la SÉQ ID N° : 14, et CDR3 ayant une séquence d'acides aminés de la SÉQ ID N° : 15 ; et
(3) un anticorps ou un fragment d'anticorps de celui-ci qui comporte une région variable d'une chaîne lourde comportant CDR1 ayant une séquence d'acides aminés de la SÉQ ID N° : 16, CDR2 ayant une séquence d'acides aminés de la SÉQ ID N° : 17, et CDR3 ayant une séquence d'acides aminés de la SÉQ ID N° : 18, et une région variable d'une chaîne légère comportant CDR1 ayant une séquence d'acides aminés de la SÉQ ID N° : 19, CDR2 ayant une séquence d'acides aminés de la SÉQ ID N° : 20, et CDR3 ayant une séquence d'acides aminés de la SÉQ ID N° : 21.

10. Kit de dosage immunologique pour détecter le SARS-CoV-2 dans un échantillon biologique, comportant deux types d'anticorps monoclonaux ou de fragments d'anticorps de ceux-ci qui se lient à un fragment de peptide ayant 30 acides aminés consécutifs ou moins dans une protéine nucléocapsidique du SARS-CoV-2, dans lequel les deux types d'anticorps monoclonaux ou de fragments d'anticorps de ceux-ci reconnaissent des épitopes différents, et
dans lequel le fragment de peptide ayant 30 acides aminés consécutifs ou moins est un fragment de peptide ayant une séquence d'acides aminés représentée par KQQTVTLLPAADLDDFSKQLQQSMSSA (SÉQ ID N° : 1).

11. Kit de dosage immunologique selon la revendication 10, dans lequel l'échantillon biologique est une sécrétion respiratoire.

12. Kit de dosage immunologique selon la revendication 10 ou 11, dans lequel les deux types d'anticorps monoclonaux ou de fragments d'anticorps de ceux-ci sont un premier anticorps monoclonal ou un fragment d'anticorps de celui-ci et un second anticorps monoclonal ou un fragment d'anticorps de celui-ci, dans lequel le premier anticorps monoclonal ou le fragment d'anticorps de celui-ci a une substance de marquage indirectement ou directement liée à celui-ci, et le second anticorps monoclonal ou le fragment d'anticorps de celui-ci est indirectement ou directement lié à une phase solide.

13. Kit de dosage immunologique selon la revendication 12, dans lequel le premier anticorps monoclonal ou le fragment d'anticorps de celui-ci est un anticorps monoclonal ou un fragment d'anticorps de celui-ci qui reconnaît un épitope dans la séquence d'acides aminés représentée par KQQTVTLLPAADLDFSK (SÉQ ID N° : 2), et le second anticorps monoclonal ou le fragment d'anticorps de celui-ci est un anticorps monoclonal ou un fragment d'anticorps de celui-ci qui reconnaît un épitope dans la séquence d'acides aminés représentée par AADLDDFSKQLQQSMSSA (SÉQ ID N° : 3).

14. Kit de dosage immunologique selon l'une quelconque des revendications 10 à 12, dans lequel l'un des anticorps monoclonaux reconnaît un épitope dans la séquence d'acides aminés représentée par KQQTVTLLPAADLDDFSK (SÉQ ID N° : 2), alors que l'autre anticorps monoclonal reconnaît un épitope dans la séquence d'acides aminés représentée par AADLDDFSKQLQQSMSSA (SÉQ ID N° : 3).

15. Kit de dosage immunologique selon l'une quelconque des revendications 10 à 13, dans lequel les deux types d'anticorps monoclonaux ou de fragments d'anticorps de ceux-ci sont, respectivement, un anticorps monoclonal ou un fragment d'anticorps de celui-ci qui reconnaît une séquence d'acides aminés représentée par LLPAA (SÉQ ID N° : 26) comme un épitope, et un anticorps monoclonal ou un fragment d'anticorps de celui-ci qui reconnaît un épitope dans une séquence d'acides aminés représentée par LDDFSKQLQ (SÉQ ID N° : 27).

16. Kit de dosage immunologique selon l'une quelconque des revendications 10 à 15, qui est un kit pour immunochromatographie ou ELISA.

17. Kit de dosage immunologique selon l'une quelconque des revendications 10 à 16, dans lequel les deux types d'anticorps monoclonaux ou de fragments d'anticorps de ceux-ci sont choisis parmi le groupe constitué de :
(1) un anticorps ou un fragment d'anticorps de celui-ci qui comporte une région variable d'une chaîne lourde comportant CDR1 ayant une séquence d'acides aminés de la SÉQ ID N° : 4, CDR2 ayant une séquence d'acides aminés de la SÉQ ID N° : 5, et CDR3 ayant une séquence d'acides aminés de la SÉQ ID N° : 6, et une région variable d'une chaîne légère comportant CDR1 ayant une séquence d'acides aminés de la SÉQ ID N° : 7, CDR2 ayant une séquence d'acides aminés de la SÉQ ID N° : 8, et CDR3 ayant une séquence d'acides aminés de la SÉQ ID N° : 9 ;
(2) un anticorps ou un fragment d'anticorps de celui-ci qui comporte une région variable d'une chaîne lourde comportant CDR1 ayant une séquence d'acides aminés de la SÉQ ID N° : 10, CDR2 ayant une séquence d'acides aminés de la SÉQ ID N° : 11, et CDR3 ayant une séquence d'acides aminés de la SÉQ ID N° : 12, et une région variable d'une chaîne légère comportant CDR1 ayant une séquence d'acides aminés de la SÉQ ID N° : 13, CDR2 ayant une séquence d'acides aminés de la SÉQ ID N° : 14, et CDR3 ayant une séquence d'acides aminés de la SÉQ ID N° : 15 ; et
(3) un anticorps ou un fragment d'anticorps de celui-ci qui comporte une région variable d'une chaîne lourde comportant CDR1 ayant une séquence d'acides aminés de la SÉQ ID N° : 16, CDR2 ayant une séquence d'acides aminés de la SÉQ ID N° : 17, et CDR3 ayant une séquence d'acides aminés de la SÉQ ID N° : 18, et une région variable d'une chaîne légère comportant CDR1 ayant une séquence d'acides aminés de la SÉQ ID N° : 19, CDR2 ayant une séquence d'acides aminés de la SÉQ ID N° : 20, et CDR3 ayant une séquence d'acides aminés de la SÉQ ID N° : 21.
